# EUROPEAN PATENT APPLICATION

(11) **EP 1 752 158 A1**
(43) Date of publication of application: **14.02.2007**
(21) Application number: 05734734.6
(22) Date of filing: 21.04.2005
(51) Int. Cl.: A61K 38/00, A61P 27/02

(54) **CORNEAL NEURITOGENESIS PROMOTER CONTAINING PACAP AND ITS DERIVATIVE**

(30) Priority: 23.04.2004 JP 2004128581; 15.11.2004 JP 2004330464
(71) Applicant: Senju Pharmaceutical Co., Ltd., Osaka-shi, Osaka 541-0046 (JP); Itoham Foods Inc., Kobe-shi, Hyogo 657-0037 (JP)
(72) Inventor: TAKAYAMA, Yoshiko;, Kobe-shi, Hyogo;6550003 (JP); NAKAMURA, Yoshikuni;, Hyogo;6530841 (JP); INOUE, Yutaka, 6650861 (JP); YABUTA, Chiho, 6540151 (JP); AZUMA, Mitsuyoshi, 6620031 (JP); ONOUE, Satomi, 4750836 (JP)
(74) Representative: Wright, Robert Gordon McRae
(86) International application number: PCT/JP2005/007609
(87) International publication number: WO 2005/102375

(57) **Abstract**

It is intended to provide an agent for promoting corneal neuritogenesis containing PACAP, a PACAP derivative or a pharmaceutically acceptable salt thereof, in particular, an agent for promoting corneal neuritogenesis aiming at improving corneal sensitivity, treating dry eye and treating corneal epithelial injury due to an effect of promoting corneal neuritogenesis. This agent for promoting corneal neuritogenesis is useful as a drug for ameliorating reduction in corneal sensitivity following corneal surgeries such as laser keratonomy (LASIK) and corneal grafting or cataract surgery, reduction in corneal sensitivity accompanying corneal neurodegeneration and dry eye symptom and corneal epithelial injury accompanying such reduction in corneal sensitivity. Moreover, it is useful as a drug for ameliorating dry eye symptom, reduction in corneal sensitivity and corneal epithelial injury in patients with dry eye, and a drug for ameliorating corneal epithelial injury and dry eye symptom and reduction in corneal sensitivity accompanying therewith.

## Description

### Technical Field

The present invention relates to an agent for promoting corneal neuritogenesis, an agent for improving corneal sensitivity, a therapeutic agent for dry eyes and a therapeutic agent for corneal epithelial injury. The present invention also relates to an agent for promoting corneal neuritogenesis useful for improving corneal sensitivity, treating dry eyes and treating corneal epithelial injury based on a corneal neuritogenesis promoting effect.

### Background Art

It is generally believed that reduction in corneal sensitivity occurs and continues for about 3 weeks to 1 year after corneal surgery such as photorefractive keratectomy (PRK), laser assisted in situ keratomileusis (LASIK) or corneal grafting because corneal nerves are cut by the surgery. For example, corneal nerves are evidently cut after LASIK (see Linna, Tuuli U et al., Experimental Eye Research, 1998, vol. 66, p755-763), and corneal sensitivity is reduced in the corneal region where nerve images are not observed or nerve fiber bundles are too short to be connected (see Linna, Tuuli U et al., Investigative Ophthalmology & Visual Sciences, 2000, vol. 41, p393-397).

It is suggested that reduction in corneal sensitivity after PRK or LASIK causes a slow response of lacrimal glands and a decrease in tear secretion (see Robert T. Ang et al., Current Opinion of Ophthalmology, 2001, vol. 12, p318-322). The reduction in corneal sensitivity function after corneal surgery causes a decrease in patient's eye blink frequency, which leads to a dry eye symptom. On the other hand, in the case of a patient with dry eyes, a decrease in tear function causes reduced corneal sensitivity, which further deteriorates the decreased tear function and consequently leads to a severe symptom of the corneal surface. In addition, the dry eye symptom caused by PRK or LASIK interferes with the wound healing of cornea (see Ang R. T. et al., Current Opinion of Ophthalmology, 2001, vol. 12, p318-322), and recurrent corneal erosion is observed after LASIK surgery (see Solomon R. et al., The Ocular Surface, 2004, vol. 2, p34-42).

However, at present, the recovery of corneal sensitivity reduced by corneal surgery is left to spontaneous recovery. Also in the treatment of dry eyes, aggressive treatment for the recovery of corneal sensitivity is not applied. Reduction in corneal sensitivity is also induced in diseases with corneal neurodegeneration such as neuroparalytic keratopathy, corneal ulcer or diabetic keratitis. However, proper therapy for reduced corneal sensitivity has not been found yet.

VIP (vasoactive intestinal peptide) is a peptide consisting of 28 amino acid residues, which was first isolated from the swine intestinal tract (see, for example, Said, S. I. et al., Science, USA, 1970, vol. 169, p1217-1218), and has a variety of biological activities including relaxation of the smooth muscles of the digestive tract and blood vessels. With respect to its effect on lacrimal glands, it has been reported that VIP promotes protein secretion from lacrimal glands (see, for example, Dartt, D. A. et al., American Journal of Physiology, 1984, vol. 247, pG502-509). Although WO 03/039577 discloses use of a VIP derivative as a dry eye treating agent, it has no description about corneal sensitivity.

PACAP (pituitary adenylate cyclase activating polypeptide) is a peptide consisting of 38 amino acid residues, which was isolated from the ovine hypothalamus in 1989 using a bioassay system for activating adenylate cyclase of hypothalamus culture cells as an index and then its structure was determined. There are two types of PACAP, that is, PACAP38 consisting of 38 amino acid residues and PACAP27 consisting of 27 N-terminal amino acid residues of PACAP38 (see, for example, Miyata A. et al., Biochemical and Biophysical Research Communications, 1989, vol. 164, p567-574). The amino acid sequence of 27 N-terminal amino acids of PACAP is quite similar to that of VIP. In addition, it is believed that VIP and PACAP belong to the glucagon-secretin superfamily because their amino acid sequences are similar to those of secretin and glucagon. Three types of PACAP receptors (PAC1, VPAC1 and VPAC2) are identified, and VPAC1 and VPAC2 receptors also have a binding affinity to VIP which is comparable with a binding affinity to PACAP (see Vaudry D. et. al., Pharmacological Review, 2000, vol. 52, p269-324). The effect of PACAP includes, in addition to relaxation of bronchial smooth muscles and dilatation of peripheral blood vessels, protection of nerve cells as a central action (see, for example, Arimura A. et al., Annuals of New York Academy of Science, 1994, vol. 739, p228-243). Although PACAP has been reported to promote cell process formation of cultured PC12 cells, which is non-nervous cells, the effect of PACAP on neither nerve cells distributed in a cornea nor secretion of tear has been reported (see, for example, Deutsch P. J. et al, The Journal of Biological Chemistry, 1992, vol. 267, p5108-5113).

Some publications disclose that a derivative synthesized based on a difference between PACAP and VIP in the amino acid sequences has a bronchodilation or hypotensive action, a blood stream increasing action, a hair restoration action, a sexual behavior activating action (see, for example, JP-A 11-100399), a digestive tract motion suppressing action (see, for example, JP-A 2001-151799) and a neuritogenesis inducing action for ameliorating Alzheimer's disease, Parkinson's disease or the like (see, for example, JP-A 2001-226284). However, they have no description showing or suggesting that said derivative has a corneal sensitivity improving effect, a lacrimal secretion promoting effect or a corneal epithelial injury treating effect.

Although WO 03/020281 discloses that a compound for promoting neuronal regeneration or axonal elongation is useful in the treatment of corneal nerve injury after LASIK surgery or the like and that an example of the compound is neotrophin, which is a neurotrophic factor stimulating substance, it has no description showing or suggesting VIP, PACAP or a derivative thereof.

### Disclosure of Invention

### Problems to be solved by the Invention

An objective of the present invention is to provide a pharmaceutical composition for ameliorating reduction in corneal sensitivity following corneal surgery such as laser assisted in situ keratomileusis (LASIK) or corneal grafting, reduction in corneal sensitivity accompanying corneal neurodegenerative diseases, and a dry eye symptom or corneal epithelial injury accompanying reduction in corneal sensitivity, and a pharmaceutical composition for ameliorating a dry eye symptom, reduced corneal sensitivity and corneal epithelial injury in a dry eye patient; and further to provide a pharmaceutical composition for ameliorating corneal epithelial injury, and a dry eye symptom and reduction in corneal sensitivity accompanying the corneal epithelial injury.

### Means for Solving the Problem

The inventors of the present invention studied in order to provide a novel type of pharmaceutical composition for ameliorating reduced corneal sensitivity following corneal surgery and reduced corneal sensitivity due to dry eyes. As a result, they found for the first time that PACAP and a novel derivative thereof whose stability in a solution state is increased (hereinafter, may be referred to as a PACAP derivative) have a neuritogenesis promoting effect in trigeminal nerve cells (hereinafter, may be referred to as corneal nerve cells) and thereby a corneal sensitivity improving effect, as well as a tear secretion promoting effect and a corneal epithelial wound healing effect. They further studied based on these findings, and as a result, completed the present invention wherein PACAP and a PACPA derivative are used as a drug for promoting corneal neuritogenesis, for improving corneal sensitivity, for treating dry eyes and for treating corneal epithelial injury.

Accordingly, the present invention relates to:
(1) an agent for promoting corneal neuritogenesis comprising PACAP, a PACAP derivative or a pharmaceutically acceptable salt thereof;
(2) an agent for improving corneal sensitivity comprising PACAP, a PACAP derivative or a pharmaceutically acceptable salt thereof;
(3) a therapeutic agent for dry eyes comprising PACAP, a PACAP derivative or a pharmaceutically acceptable salt thereof;
(4) a therapeutic agent for corneal epithelial injury comprising PACAP, a PACAP derivative or a pharmaceutically acceptable salt thereof;
(5) the agent for promoting corneal neuritogenesis according to the above (1), wherein the PACAP derivative is a peptide (SEQ ID Nos. 1 to 14) comprising at least 23 residues from the N-terminal of a peptide represented by the following formula (I):

   His-Ser-Asp-Ala-X1-Phe-Thr-X2-X3-Tyr-X4-Arg-X5-Arg-X6-Gln-X7-Ala-Val-X8-X9-Tyr-Leu-Ala-Ala-X10-X11-X12 (I)

   wherein,
   X1 represents Val or Ile;
   X2 represents Asp, Ala or Glu;
   X3 represents Asn or Ser;
   X4 represents Thr or Ser;
   X5 represents Leu or Tyr;

   X6, X8 and X9 represent Lys or Arg;
   X7 represents Leu or Nle;
   X10 represents Ile, Val or a chemical bond;
   X11 represents Leu, Leu-Asn, Leu-Gly, Leu-Gly-Lys, Leu-Gly-Arg, Leu-Gly-Lys-Lys, Leu-Gly-Lys-Arg, Leu-Gly-Arg-Arg, Leu-Gly-Lys-Arg-Tyr-Lys-Gln-Arg-Val-Lys-Asn-Lys, Leu-Gly-Arg-Arg-Tyr-Arg-Gln-Arg-Val-Arg-Asn-Arg, or a chemical bond; and
   X12 modifies the α-carboxyl group of the C-terminal amino acid, and represents -NH₂ or -OH; or pharmaceutically acceptable salts thereof;
(6) the agent for improving corneal sensitivity according to the above (2), wherein the PACAP derivative is a peptide comprising at least 23 residues from the N-terminal of a peptide represented by the above formula (I) or a pharmaceutically acceptable salt thereof;
(7) the therapeutic agent for dry eyes according to the above (3), wherein the PACAP derivative is a peptide comprising at least 23 residues from the N-terminal of a peptide represented by the above formula (I) or a pharmaceutically acceptable salt thereof;
(8) the therapeutic agent for corneal epithelial injury according to the above (4), wherein the PACAP derivative is a peptide comprising at least 23 residues from the N-terminal of a peptide represented by the above formula (I) or a pharmaceutically acceptable salt thereof;
(9) the agent for promoting corneal neuritogenesis according to the above (5), wherein the PACAP derivative is a peptide represented by the above formula (I) in which X1 is Ile, X2 is Asp, X3 is Ser, X4 is Ser, X5 is Tyr, X6, X8 and X9 are Arg, X7 is Leu, X10 is Val and X11 is Leu-Gly-Arg-Arg;
(10) the agent for improving corneal sensitivity according to the above (6), wherein the PACAP derivative is a peptide represented by the above formula (I) in which X1 is Ile, X2 is Asp, X3 is Ser, X4 is Ser, X5 is Tyr, X6, X8 and X9 are Arg, X7 is Leu, X10 is Val and X11 is Leu-Gly-Arg-Arg;
(11) the therapeutic agent for dry eyes according to the above (7), wherein the PACAP derivative is a peptide represented by the above formula (I) in which X1 is Ile, X2 is Asp, X3 is Ser, X4 is Ser, X5 is Tyr, X6, X8 and X9 are Arg, X7 is Leu, X10 is Val and X11 is Leu-Gly-Arg-Arg;
(12) the therapeutic agent for corneal injury according to the above (8), wherein the PACAP derivative is a peptide represented by the above formula (I) in which X1 is Ile, X2 is Asp, X3 is Ser, X4 is Ser, X5 is Tyr, X6, X8 and X9 are Arg, X7 is Leu, X10 is Val and X11 is Leu-Gly-Arg-Arg;
(13) use of PACAP, a PACAP derivative or a pharmaceutically acceptable salt thereof in the production of an agent for promoting corneal neuritogenesis;
(14) the use according to the above (13), wherein the PACAP derivative is a peptide comprising at least 23 residues from the N-terminal of a peptide represented by the above formula (I) or a pharmaceutically acceptable salt thereof;
(15) use of PACAP, a PACAP derivative or a pharmaceutically acceptable salt thereof in the production of an agent for improving corneal sensitivity;
(16) the use according to the above (15), wherein the PACAP derivative is a peptide comprising at least 23 residues from the N-terminal of a peptide represented by the above formula (I) or a pharmaceutically acceptable salt thereof;
(17) use of PACAP, a PACAP derivative or a pharmaceutically acceptable salt thereof in the production of a therapeutic agent for dry eyes;
(18) the use according to the above (17), wherein the PACAP derivative is a peptide comprising at least 23 residues from the N-terminal of a peptide represented by the above formula (I) or a pharmaceutically acceptable salt thereof;
(19) use of PACAP, a PACAP derivative or a pharmaceutically acceptable salt thereof in the production of a therapeutic agent for corneal epithelial injury;
(20) the use according to the above (19), wherein the PACAP derivative is a peptide comprising at least 23 residues from the N-terminal of a peptide represented by the above formula (I) or a pharmaceutically acceptable salt thereof;
(21) a method for promoting corneal neuritogenesis which comprises administering an effective amount of PACAP, a PACAP derivative or a pharmaceutically acceptable salt thereof to a subject in need thereof;
(22) the method according to the above (21), wherein the PACAP derivative is a peptide comprising at least 23 residues from the N-terminal of a peptide represented by the above formula (I) or a pharmaceutically acceptable salt thereof;
(23) a method for improving corneal sensitivity which comprises administering an effective amount of PACAP, a PACAP derivative or a pharmaceutically acceptable salt thereof to a subject in need thereof;
(24) the method according to the above (23), wherein the PACAP derivative is a peptide comprising at least 23 residues from the N-terminal of a peptide represented by the above formula (I) or a pharmaceutically acceptable salt thereof;
(25) a method for treating dry eyes which comprises administering an effective amount of PACAP, a PACAP derivative or a pharmaceutically acceptable salt thereof to a subject in need thereof;
(26) the method according to the above (25), wherein the PACAP derivative is a peptide comprising at least 23 residues from the N-terminal of a peptide represented by the above formula (I) or a pharmaceutically acceptable salt thereof;
(27) a method for treating corneal epithelial injury which comprises administering an effective amount of PACAP, a PACAP derivative or a pharmaceutically acceptable salt thereof to a subject in need thereof;
(28) the method according to the above (27), wherein the PACAP derivative is a peptide comprising at least 23 residues from the N-terminal of a peptide represented by the above formula (I) or a pharmaceutically acceptable salt thereof; and the like.

As used herein, the "corneal nerve" is under the control of the trigeminal nerve, which is a sensory nerve, and refers to the ring-like neuroplexus formed around the cornea, the intrastromal neuroplexus distributed in a net-like pattern in the corneal stroma, the subepithelial neuroplexus formed beneath the Bowman membrane, the basal cell neuroplexus formed on a place across the Bowman membrane, and nerve fibers. As used herein, the "neurite" refers to processes (dendrites and axons) protruding from neurons (nerve cells), and the "genesis" means that the neurite as defined above is formed and/or elongated on the cells.

### Effect of the invention

Since the pharmaceutical composition of the present invention which comprises PACAP or a PACAP derivative has a promoting effect on neuritogenesis in trigeminal nerve cells, a corneal sensitivity improving effect, a tear secretion promoting effect and a corneal epithelial wound healing promoting effect, it is useful in (1) improving corneal sensitivity reduced by injury of corneal nerves or the like and ameliorating a dry eye symptom and corneal epithelial injury accompanying reduction in corneal sensitivity; (2) ameliorating a dry eye symptom, reduced corneal sensitivity and corneal epithelial injury in dry eye patients; and (3) ameliorating corneal epithelial injury, and reduction in corneal sensitivity and a dry eye symptom accompanying the corneal epithelial injury.

### Brief Description of Drawings

Fig. 1 shows fluorescence microscope images of cultured rabbit trigeminal nerve cells in Test Example 1. Cells of (A) non-addition group, (B) PACAP27 (1 µM) addition group, and (C) peptide 17 (1 µM) addition group are shown. Bar = 100 µm

Fig. 2 shows the proportion (%) of neurite forming cells in the total number of cells in Test Example 1. The vertical axis shows the proportion of neurite forming cells in the total cells. The mark *** in the graph shows a significant difference (p<0.001) relative to the non-addition group.

Fig. 3 shows time-dependent changes in the corneal sensitivity of rabbits in Test Example 2. The marks * and ** in the graph show significant differences (*: p<0.05; **: p<0.005) relative to the control group.

Fig. 4 shows time-dependent changes in the protein secretion rates of lacrimal glands isolated from rabbits in Test Example 3. The mark * in the graph shows a significant difference (p<0.05) relative to the control group.

Fig. 5 shows fluorescence microscope images of cultured rabbit trigeminal nerve cells in Test Example 4. Cells of (A) non-addition group, (B) PACAP27 (1 µM) addition group and (C) peptide 24 (1 uM) addition group. Bar = 100 µm

Fig. 6 shows neuritogenesis promoting effects of PACAP27 and peptide 24 examined in Test Example 4. The vertical axis shows the proportion (%) of neurite forming cells in the total cells. The mark * in the graph shows a significant difference (p<0.05, three samples in each group, average value ± standard deviation) relative to the non-addition group.

Fig. 7 shows the secretion rates of protein from lacrimal glands isolated from rabbits in Test Example 5. The mark * in the graph shows a significant difference (p<0.05, three samples in each group, average value ± standard deviation) relative to the non-addition group.

Fig. 8 shows time-dependent changes in the secretion amount of rabbit tear in Test Example 6. The mark * in the graph shows a significant difference (p<0.05, 10 samples in each group, average value ± standard deviation) relative to the non-addition group.

Fig. 9 shows changes in corneal sensitivity before and after cutting the rabbit corneal nerve in Test Example 7. The vertical axis shows the length of a nylon filament in an aesthesiometer used for measuring corneal sensitivity. The horizontal axis shows the lapse time (weeks) after creating corneal flaps (cutting the corneal nerve). Each measured value is shown as an average value (8 samples in the control group, and 7 samples in the group using eye drops of peptide 24) ± standard deviation. The mark * in the graph shows a significant difference (p<0.05) relative to the control group.

Fig. 10 shows proliferation of cultured human corneal epithelial cells when peptide 24 or PACAP27 is added to the cells in Test Example 8. The vertical axis shows the proportion (%) of the cell number of the test substance addition group relative to the cell number of the control group. The mark * in the graph shows a significant difference relative to the control group (average values ± standard error, n = 8 to 16, p<0.05).

Fig. 11 shows changes in the wound healing of rabbit cornea in Test Example 9. The vertical axis shows the remaining (%) of the corneal epithelium defect area. The horizontal axis shows time (hours). Groups using 10 µM and 100 µM PACAP27 as eye drops showed significant differences relative to the control group (* p<0.05 vs. control group, Dunnet-test, average value ± standard deviation), N = 4: control group and 100 µM PACAP27 group, N = 5: 10 µM PACAP27 group).

### Best Mode for Carrying Out the Invention

Of peptides used in the present invention, PACAP is PACAP27 (SEQ ID No. 15) or PACAP38 (SEQ ID No. 16) peptide. A derivative of PACAP is a peptide prepared by substituting a part of the amino acids constituting PACAP with other amino acids, by inserting other amino acids into the amino acid sequence of PACAP, or by deleting a part of the amino acids of PACAP. Specifically, the peptide preferably comprises the amino acid sequence represented by the formula (I) or a pharmaceutically acceptable salt thereof. The N-terminal of the peptide represented by the formula (I) may be an amino group, or may be chemically modified if necessary. Since the smallest active unit of PACAP (Kitada, C. et al., Peptide Chemistry, 1990, 239-244, 1991) or VIP (Onoue, S. et al., Eur. Journal Pharamacol., 485:307-16, 2004) comprises 23 residues from the N-terminal, the PACAP derivative of the present invention has to retain at least 23 residues from the N-terminal. Representative examples of the peptide represented by the formula (I) are shown in Table 1. Peptide Nos. 1 to 24 shown herein correspond to peptides shown in SEQ ID Nos. 17 to 40; provided that peptide Nos. 1, 13, 15, 22 and 23 are peptides consisting of 23 residues from the N-terminal, an acetyl group is linked to the N-terminal amino groups of peptide Nos. 4, 19 and 23, and a stearoyl group is linked to the N-terminal amino group of peptide No. 5. Among these peptides, preferred are peptide Nos. 1, 3, 12, 14, 16, 17, 20, 22 and 24, and more preferred are peptide Nos. 17 and 24.

**[Table 1]**

| * | X1 | X2 | X3 | X4 | X5 | X6 | X7 | X8 | X9 | X10 | X11 | X12 |
|---|---|---|---|---|---|---|---|---|---|---|---|---|
| 1 | Val | Asp | Asn | Thr | Leu | Arg | Leu | Arg | Arg | - | - | NH₂ |
| 2 | Val | Asp | Asn | Thr | Leu | Lys | Leu | Lys | Lys | Ile | Leu-Asn | NH₂ |
| 3 | Val | Asp | Asn | Thr | Leu | Arg | Leu | Arg | Arg | Ile | Leu-Gly-Arg-Arg | NH₂ |
| 4 | Val | Asp | Asn | Thr | Leu | Arg | Leu | Arg | Arg | Ile | Leu-Gly-Arg-Arg | NH₂ |
| 5 | Val | Asp | Asn | Thr | Leu | Arg | Leu | Arg | Arg | Ile | Leu-Gly-Arg-Arg | NH₂ |
| 6 | Val | Asp | Asn | Thr | Leu | Arg | Leu | Arg | Arg | Ile | Leu-Gly-Arg-Arg | OH |
| 7 | Val | Asp | Asn | Thr | Leu | Arg | Nle | Arg | Arg | Ile | Leu-Gly-Arg-Arg | NH₂ |
| 8 | Val | Asp | Asn | Thr | Leu | Arg | Leu | Arg | Arg | Ile | Leu-Gly | NH₂ |
| 9 | Val | Asp | Asn | Thr | Leu | Arg | Leu | Arg | Arg | Ile | Leu-Gly-Lys | NH₂ |
| 10 | Val | Asp | Asn | Thr | Leu | Arg | Leu | Arg | Arg | Ile | Leu-Gly-Arg | NH₂ |
| 11 | Val | Asp | Asn | Thr | Leu | Arg | Leu | Arg | Arg | Ile | Leu-Gly-Lys-Arg | NH₂ |
| 12 | Val | Glu | Asn | Thr | Leu | Arg | Leu | Arg | Arg | Ile | Leu-Gly-Arg-Arg | NH₂ |
| 13 | Val | Glu | Asn | Thr | Leu | Arg | Leu | Arg | Arg | - | - | NH₂ |
| 14 | Val | Ala | Asn | Thr | Leu | Arg | Leu | Arg | Arg | Ile | Leu-Gly-Arg-Arg | NH₂ |
| 15 | Val | Ala | Asn | Thr | Leu | Arg | Leu | Arg | Arg | - | - | NH₂ |
| 16 | Val | Asp | Asn | Thr | Leu | Arg | Leu | Arg | Arg | Val | Leu-Gly-Arg-Arg | NH₂ |
| 17 | Ile | Asp | Ser | Ser | Tyr | Arg | Leu | Arg | Arg | Val | Leu-Gly-Arg-Arg | OH |
| 18 | Ile | Asp | Ser | Ser | Tyr | Arg | Leu | Arg | Arg | Ile | Leu-Gly-Arg-Arg | NH₂ |
| 19 | Ile | Asp | Ser | Ser | Tyr | Arg | Leu | Arg | Arg | Val | Leu-Gly-Arg-Arg | NH₂ |
| 20 | Ile | Asp | Ser | Ser | Tyr | Arg | Leu | Arg | Arg | Val | Leu-Gly-Arg-Arg - Tyr-Arg-Gln-Arg - Val-Arg-Asn-Arg | NH₂ |
| 21 | Ile | Asp | Ser | Ser | Tyr | Arg | Leu | Arg | Arg | Ile | Leu-Gly-Arg-Arg - Tyr-Arg-Gln-Arg - Val-Arg-Asn-Arg | NH₂ |
| 22 | Ile | Asp | Ser | Ser | Tyr | Arg | Leu | Arg | Arg | - | - | NH₂ |
| 23 | Ile | Asp | Ser | Ser | Tyr | Arg | Leu | Arg | Arg | - | - | NH₂ |
| 24 | Ile | Asp | Ser | Ser | Tyr | Arg | Leu | Arg | Arg | Val | Leu-Gly-Arg-Arg | NH₂ |

| | | | | | | | | | | | | |
|---|---|---|---|---|---|---|---|---|---|---|---|---|
| *: peptide No. His-Ser-Asp-Ala-X1-Phe-Thr-X2-X3-Tyr-X4-Arg-X5-Arg-X6-Gln-X7-Ala-Val-X8-X9-Tyr-Leu-Ala-Ala-X10-X11-X12 (I) (wherein X12 represents α-carboxyl group modification of the C-terminal amino acid.) The peptide comprising at least 23 residues from the N-terminal of a peptide represented by the formula (I) is represented by the formula: His-Ser-Asp-Ala-X1-Phe-Thr-X2-X3-Tyr-X4-Arg-X5-Arg-X6-Gln-X7-Ala-Val-X8-X9-Tyr-Leu-X13 (I') (wherein X13 represents X12, Ala-X12 or Ala-Ala-X10-X11-X12). | | | | | | | | | | | | |

Examples of a pharmaceutically acceptable salt of the compound of the present invention include salts with alkali metals such as sodium and potassium; salts with alkali earth metals such as calcium and magnesium; salts with inorganic bases such as aluminum salts and ammonium salts; salts with organic bases such as trimethylamine, pyridine, picoline, ethanolamine, diethanolamine, triethanolamine, dicyclohexylamine and N,N-dibenzylethylenediamine; salts with inorganic acids such as hydrochloric acid, hydrobromic acid, nitric acid, sulfuric acid and phosphoric acid; salts with organic acids such as formic acid, acetic acid, trifluoroacetic acid, fumaric acid, oxalic acid, tartaric acid, lactic acid, maleic acid, citric acid, succinic acid, malic acid, methanesulfonic acid, benzenesulfonic acid and p-toluenesulfonic acid; and salts with polymerized acids such as tannic acid, carboxymethyl cellulose, polylactic acid and polyglycolic acid, and the like.

The peptide used in the present invention can be synthesized by a conventional known peptide synthesis method, for example, according to description in "The Peptides", vol. 1, 1966, Schreder and Luhke, Academic Press, New York, U.S.A. or "Peptide Synthesis", Izumiya et. al., Maruzen Co., 1975, including, but not limited to, an azide method, an acid chloride method, an acid anhydride method, a mixed acid anhydride method, a DCC method, an active ester method (e.g. a p-nitrophenyl ester method, an N-hydroxysuccinimide ester method, a cyanomethyl ester method, or the like), a method using Woodward's reagent K, a carboimidazole method, an oxidation-reduction method, and a DCC-additive (HONB, HOBt, HOSu) method. The above-mentioned methods can be applied to a solid state synthesis or a liquid phase synthesis.

In the present invention, peptide synthesis is accomplished by the above-mentioned conventional polypeptide synthesis method, for example by a so-called stepwise method which comprises condensing amino acids one-by-one sequentially to the terminal amino acid, or by a method which comprises coupling several peptide fragments.

A solid phase synthesis can be carried out by a stepwise method according to the Merrifield method ( Merrifield, R. B., Solid Phase Peptide Synthesis, J. Amer. Chem. Soc., 85, 2149-2159, 1963) as follows. First, a C-terminal amino acid (whose amino group is protected) is bound to an insoluble resin through its carboxyl group. Then, the protecting group of the amino group of the C-terminal amino acid is removed. To the free reactive amino group thus obtained, the reactive carboxyl group of an amino acid whose amino group is protected is coupled by a condensation reaction in accordance with the amino acid sequence of the desired peptide. After in this way the entire sequence is synthesized step-by-step, the synthesized peptide is detached from the insoluble resin.

Any insoluble resin may be used in the above-described solid phase synthesis so long as the reactive carboxyl group is able to link to the insoluble resin. Examples of the insoluble resin include a benzhydrylamine resin (BHA resin), a chloromethyl resin, an oxymethyl resin, an aminomethyl resin, a methylbenzhydryl resin (MBHA resin), a 4-aminomethylphenoxymethyl resin, a 4-hydroxymethylphenoxymethyl resin and a 4-oxymethylphenylacetamidomethyl resin.

When a 9-fluorenylmethyloxycarbonyl group (Fmoc) is used as a protecting group for the α-amino group, a resin from which a peptide can be detached with trifluoroacetic acid (TFA), for example, a 4-hydroxymethylphenoxymethyl resin is preferably used. When a t-butoxycarbonyl group (Boc) is used as a protecting group for the α-amino group, a resin from which a peptide can be detached with hydrogen fluoride, for example, a 4-oxymethylphenylacetamidomethyl resin (PAM resin) is preferably used. The amount of a peptide per 1 g of a resin is preferably 0.5 mmol or less.

The above-described method requires coupling of a protecting group to the amino group which is involved in a peptide bond between amino acids, elimination of the protecting group, and activation of the carboxyl group which is involved in a peptide bond between amino acids.

Examples of a protecting group for the amino group include benzyloxycarbonyl (Z), t-butoxycarbonyl (Boc), t-aminooxycarbonyl (Aoc), isobonyloxycarbonyl, p-methoxybenzyloxycarbonyl, 2-chloro-benzyloxycarbonyl, adamantyloxycarbonyl, trifluoroacetyl, phthaloyl, formyl, o-nitrophenylsulfenyl and diphenylphosphinothioyl.

In the case of amino acids having a functional group at their side chains, such as His, Tyr, Thr, Lys, Asp, Arg and Ser, the functional group at the side chain is preferably protected. Protection of the functional group is accomplished by attaching a conventional protecting group mentioned below according to a conventional method. The protecting group is eliminated after the reaction.

Examples of a protecting group for the imino group of His include benzyloxymethyl (Bom), p-toluenesulfonyl (Tos), benzyl (Bzl), benzyloxycarbonyl (Z), and trityl.

Although the hydroxyl groups of Ser and Thr may be protected by esterification or etherification, the protection is not essential. Examples of a group suitable for esterification include a lower alkanoyl group such as an acetyl group, an aroyl group such as a benzoyl group, and a group derived from carbonic acid such as benzoyloxycarbonyl or ethyloxycarbonyl. Examples of a group suitable for etherification include benzyl (Bzl), tetrahydropyranyl and tert-butyl.

Examples of a protecting group for the hydroxyl group of Tyr include benzyl (Bzl), bromobenzyloxycarbonyl (Br-Z), dichlorobenzyl (Cl₂-Bzl), benzyloxycarbonyl (Z), acetyl, and p-toluenesulfonyl (Tos).

Examples of a protecting group for the amino group of Lys include benzyloxycarbonyl (Z), chlorobenzyloxycarbonyl (Cl-Z), dichlorobenzyl (Cl₂-Bzl), t-butoxycarbonyl (Boc), and p-toluenesulfonyl (Tos).

Examples of a protecting group for the guanidino group of Arg include p-toluenesulfonyl (Tos), nitro, benzyloxycarbonyl (Z), and t-amyloxycarbonyl (Aoc).

The carboxyl group of Asp is protected by esterification, for example, with benzyl alcohol, methanol, ethanol, tert-butanol, cyclohexyl (cHex) or the like.

Examples of a protecting group for the indolyl group of Trp include formyl, carbobenzoxyl, 4-methoxy-2,3,6-trimethylbenzenesulfonyl and 2,2,2-trichloroethyloxycarbonyl, but the protection is not essential.

The thiomethyl group of Met may be protected by conversion into methylsulfoxide followed by reduction, but the protection is not essential.

The carboxyl group can be activated by a conventional method, and reagents used in the method may be appropriately selected from known reagents. For example, the carboxyl group is activated by reacting the carboxyl group with various reagents to form the corresponding acid chloride, acid anhydride or mixed acid anhydride, azide, activated ester (ester with pentachlorophenol, p-nitrophenol, N-hydroxysuccinimide, N-hydroxybenzotriazole, N-hydroxy-5-norbornene-2,3-dicarboxyimide or the like) or the like.

Any solvent may be used in the above-described solid phase condensation reaction (peptide bond forming reaction) between a reactive amino group and a reactive carboxyl group so long as the solvent can be used for peptide bond formation. Examples of the solvent include anhydrous or hydrated dimethylformamide (DMF), dimethylsulfoxide (DMSO), pyridine, chloroform, dioxane, dichloromethane (DCM), tetrahydrofuran (THF), ethyl acetate, N-methylpyrrolidone and hexamethylphosphoric acid triamide (HMPA) as a single solvent or as a mixed solvent of two or more of them.

The above-described condensation reaction may be carried out in the presence of a condensing agent, for example, a carbodiimide reagent such as dicyclohexylcarbodiimide (DCC) or carbodiimidazole, tetraethylpyrophosphate, benzotriazol-N-hydroxytrisdimethylaminophosphonium hexafluorophosphate (Bop regent), or the like.

The synthesized peptide can be desalted and purified by a conventional method, for example, ion-exchange chromatography on DEAE-cellulose or the like, partition chromatography on Sephadex LH-20, Sephadex G-25 or the like, normal phase chromatography on silica gel or the like, reversed phase chromatography on ODS-silica gel or the like, high performance chromatography or the like.

The pharmaceutical composition of the present invention is useful as a remedy for promoting corneal neuritogenesis and improving reduced corneal sensitivity, a dry eye symptom and corneal epithelial injury in mammals (for example human, monkey, rat, mouse, rabbit, bovine, swine, canine and feline) and birds (fowl, pigeon and turkey). In other words, the pharmaceutical composition of the present invention is useful as a remedy for improving corneal sensitivity reduced by injury, incision or defect of corneal nerves, and for ameliorating a dry eye symptom and corneal epithelial injury accompanying reducition in corneal sensitivity. In addition, the pharmaceutical composition of the present invention is useful as a remedy for ameliorating a dry eye symptom and corneal injury which occur independently of corneal sensitivity failure. For example, the pharmaceutical composition of the present invention is useful as a remedy for improving corneal sensitivity function reduced by corneal surgery such as cataract surgery, PRK, LASIK, LASEK (laser epithelial keratomileusis; Jatta Horwath-Winter et al., J. Cataract Refract Surg., 30:2316-2321, 2004) or corneal grafting; as a remedy for improving reduced corneal sensitivity function associated with corneal neurodegenerative diseases including neuroparalytic keratitis, corneal ulcer and diabetic keratopathy; and as a treating agent for ameliorating dry eyes and corneal epithelial injury accompanying reduction in corneal sensitivity. The pharmaceutical composition of the present invention is also useful as a remedy for ameliorating a dry eye symptom in a patient with lacrimal deficiency, Sjögren syndrome, keratoconjunctivitis sicca or Steven-Johnson syndrome; and as a remedy for ameliorating a dry eye symptom associated with diseases including corneal epithelial erosion, marginal blepharitis, ocular pemphigus, vernal catarrh and allergic conjunctivitis or VDT (visual display terminal) work, and reduced corneal sensitivity and corneal epithelial injury accompanying the dry eye symptom. The pharmaceutical composition of the present invention is also useful as a remedy for ameliorating corneal epithelial injury associated with corneal epithelial erosion, marginal blepharitis, ocular pemphigus, vernal catarrh, allergic conjunctivitis or the like, and a dry eye symptom and reduced corneal sensitivity accompanying corneal epithelial injury.

The pharmaceutical composition of the present invention is systemically or topically administered. Systemic administration includes oral administration as well as parenteral administration including intravenous injection, subcutaneous injection and intramuscular injection. Topical administration is carried out by ocular instillation.

The pharmaceutical composition of the present invention can be formulated into a dosage form including a solid form such as powder, granule, tablet, capsule or suppository, and a liquid form such as syrup, injection or eye drop. For a granule and a tablet, the desired dosage form can be produced using an excipient (e.g. lactose, sucrose, glucose, starch, crystalline cellulose or the like), a lubricant (e.g. magnesium stearate, talc, stearic acid, calcium stearate or the like), a disintegrant (e.g. starch, carmellose sodium, calcium carbonate or the like), a binder (e.g. starch paste, hydroxypropyl cellulose solution, carmellose solution, gum arabic solution, gelatin solution, sodium alginate solution or the like) and the like. The granule and tablet may be coated with an appropriate coating agent (e.g. gelatin, sucrose, gum arabic, carnauba wax or the like), an enteric coating agent (e.g. cellulose acetate phthalate, methacrylate copolymer, hyroxypropyl cellulose phthalate, carboxymethylethyl cellulose or the like), or the like.

A capsule may be produced by adding appropriate excipients, for example magnesium stearate, calcium stearate, talc or light silicic anhydride for improving fluidity and lubricity, and crystalline cellulose or lactose for giving pressurized fluidity, the above-mentioned excipient and the like to the compound of the present inevntion; mixing or granulating homogeneously them; coating the resulting granules with a appropriate coating agent, if necessary; and then filling a capsules shell with the resulting mixture or granules. Alternatively, a capsule may be produced by encapsulating the above-described mixture or granules with the capsule base whose plasticity is increased by adding glycerin, sorbitol or the like to an appropriate capsule base (e.g. gelatin). Such a capsule may contain a colorant, a preservative [e.g. sulfur dioxide, or paraben (methyl, ethyl or propyl parahydroxybenzoate)] and the like, if necessary. Such a capsule may be a usual capsule or may be an enteric coating capsule, an endogastric resistant capsule or a controlled-release capsule. An enteric capsule can be produced by coating a compound with an enteric coating agent, mixing the compound with the above-mentioned excipient if necessary, and then filling a usual capsule with the resulting compound or mixture. Alternatively, the capsule itself may be coated with an enteric coating agent, or the capsule may be formed using an enteric polymer as a capsule base.

A suppository can be produced using an appropriately selected suppository base (e.g. cacao butter, macrogol or the like).

Syrup can be produced using a stabilizer (e.g. sodium edetate or the like), a suspending agent (e.g. gum arabic, carmellose or the like), a corrigent (e.g. simple syrup, glucose or the like), a flavor and the like which are appropriately selected.

When the pharmaceutical composition of the present invention is formulated into an injection or eye drops, the desired dosage form can be produced by dissolving or dispersing the compound of the present invention in a solution containing appropriately a pharmaceutically acceptable additives, for example, an isotonicity (e.g. sodium chloride, potassium chloride, glycerin, mannitol, sorbitol, erythritol, boric acid, borax, glucose, propyleneglycol or the like), a buffering agent (e.g. phosphate buffer, acetate buffer, borate buffer, carbonate buffer, citrate buffer, tris buffer, glutamate buffer, ε-aminocaproate buffer or the like), a preservative (e.g. parahydroxybenzoic acid esters, chlorobutanol, benzyl alcohol, benzarconium chloride, sodium dehydroacetate, sodium edetate, boric acid, borax or the like), a thickening agent (e.g. hydroxyethyl cellulose, hydroxypropyl cellulose, polyvinyl alcohol, polyethyleneglycol, carboxymethyl cellulose or the like), a stabilizer (e.g. sodium hydrogen sulfite, sodium thiosulfate, sodim edatate, sodium citrate, ascorbic acid, dibutylhydroxytoluene or the like), a pH adjuster (e.g. hydrochloric acid, sodium hydroxide, phosphoric acid, acetic acid or the like) and the like.

The amount of the additive contained in the syrup, injection or eye drops varies depending on the kind and purpose of the additive to be added and the like, and the additive is added in such an amount as reaches a concentration sufficient to accomplish the purpose of the additive. About 0.5 to about 5.0 w/v% of an isotonicity is usually contained in the syrup, injection or eye drops so that the osmotic pressure becomes about 229 to about 343 mOsm. About 0.01 to about 2.0 w/v% of a buffering agent is contained in the syrup, injection or eye drops. About 0.01 to about 1.0 w/v% of a thickening agent is contained in the syrup, injection or eye drops. About 0.001 to about 1.0 w/v% of a stabilizer is contained in the syrup, injection or eye drops. A pH adjuster is added as appropriate so that the pH of the syrup, injection or eye drops becomes usually about pH 3 to about pH 9, preferably about pH 4 to about pH 8.

In particular, in the case of the eye drops, the peptide concentration in the eye drops is usually at least about 0.00001 w/v%, about 0.00005 w/v% or 0.0001 w/v%, and at most about 0.1 w/v%, about 0.05 w/v%, about 0.01 w/v%, about 0.005 w/v% or about 0.001 w/v%.

In the present invention, the dose of the peptide varies depending on the disease and symptom to be treated, the subject to be administered, the administration method, and the like. For example, when an agent for improving corneal sensitivity is topically administered into the eyes of an adult patient after LASIK surgery, the eye drops containing about 0.001 w/v% of the peptide may be administered several times per day in an amount of about 20 to about 50 µL per time.

The pharmaceutical composition of the present invention can be also used in combination with other pharmaceutically effective ingredients so long as they do not disturb the objective of the present invention.

The present invention will be further explained in detail by reference to the following Examples to which the present invention is limited in any way.

### Example 1

### Synthesis of peptide

Peptide 3 having the amino acid sequence shown in SEQ ID No. 19 was produced by a conventional method for solid phase peptide synthesis.

MBHA resin HC1 salt (polystyrene-1% divinylbenzene copolymer, 100 to 200 mesh) was put into a manual synthesis reactor (made of glass, 6.0 cm (diameter) × 29.5 cm (height)), washed with methanol 2 to 3 times the volume of the resin with stirring, and then washed with dichloromethane (2 to 3 times the volume of the resin) with stirring to swell the resin. The resin was neutralized with 10% triethylamine in dichloromethane, and was condensed with about two equivalent of Boc-Arg(Tos)-OH corresponding to the C-terminal amino acid by using dicyclohexylcarbodiimide and N-hydroxybenzotriazole. After the condensation reaction was performed for about 2 hours (with stirring), the resin was washed with methanol and dichloromethane. After the disappearance of the α-amino group was confirmed by a Kaiser test, the resin was treated with 50% trifluoroacetic acid in dichloromethane for 30 minutes to deprotect the α-amino group. Then, the resin was neutralized with 10% triethylamine in dichloromethane, and washed with methanol and dichloromethane again. Subsequently, the deprotection was confirmed by a Kaiser test. After that, the same procedure as described above was repeated to couple Boc-Arg(Tos)-OH which is the second amino acid from the C-terminal to the resin. Subsequently, the coupling/deprotection reactions of Boc-Gly-OH, Boc-Leu-OH, Boc-Ile-OH, Boc-Ala-OH, Boc-Ala-OH, Boc-Leu-OH, Boc-Tyr (Cl₂-Bzl)-OH, Boc-Arg (Tos)-OH, Boc-Arg (Tos) -OH, Boc-Val-OH, Boc-Ala-OH, Boc-Leu-OH, Boc-Gln(Xan)-OH, Boc-Arg(Tos)-OH, Boc-Arg(Tos)-OH, Boc-Leu-OH, Boc-Arg(Tos)-OH, Boc-Thr(Bzl)-OH, Boc-Tyr(Cl₂-Bzl)-OH, Boc-Asn(Xan)-OH, Boc-Asp(OcHex)-OH, Boc-Thr(Bzl)-OH, Boc-Phe-OH, Boc-Val-OH, Boc-Ala-OH, Boc-Asp(OcHex)-OH, Boc-Ser(Bzl)-OH and Boc-His(Bom)-OH were performed in this order to obtain a protected peptide corresponding to peptide 3: His(Bom)-Ser(Bzl)-Asp(OcHex)-Ala-Val-Phe-Thr(Bzl)-Asp(OcHex)-Asn-Tyr(Cl₂-Bzl)-Thr(Bzl)-Arg(Tos)-Leu-Arg(Tos)-Arg(Tos)-Gln-Leu-Ala-Val-Arg(Tos)-Arg(Tos)-Tyr(Cl₂-Bzl)-Leu-Ala-Ala-Ile-Leu-Gly-Arg(Tos)-Arg(Tos)-MBHA. The protected peptide-MBHA resin thus obtained was reacted with anhydrous hydrogen fluoride in the presence of ethanedithiol and anisole. After the reaction, anhydrous hydrogen fluoride was removed by evaporation in vacuum. After the residue was washed with ether, 10% acetic acid was added to the residue to extract the peptide. The extract solution was purified by reversed phase column chromatography and then lyophilized to obtain peptide 3.

The following PACAP27, PACAP38 and peptides 1 to 24 were chemically synthesized in the same way as described above.
PACAP27 (SEQ ID No. 15):
His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu-NH₂
PACAP38 (SEQ ID No. 16):
His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu-Gly-Lys-Arg-Tyr-Lys-Gln-Arg-Val-Lys-Asn-Lys-NH₂
Peptide 1 (SEQ ID No. 17):
His-Ser-Asp-Ala-Val-Phe-Thr-Asp-Asn-Tyr-Thr-Arg-Leu-Arg-Arg-Gln-Leu-Ala-Val-Arg-Arg-Tyr-Leu-NH₂
Peptide 2 (SEQ ID Nos. 18):
His-Ser-Asp-Ala-Val-Phe-Thr-Asp-Asn-Tyr-Thr-Arg-Leu-Arg-Lys-Gln-Leu-Ala-Val-Lys-Lys-Tyr-Leu-Ala-Ala-Ile-Leu-Asn-NH₂
Peptide 3 (SEQ ID No. 19):
His-Ser-Asp-Ala-Val-Phe-Thr-Asp-Asn-Tyr-Thr-Arg-Leu-Arg-Arg-Gln-Leu-Ala-Val-Arg-Arg-Tyr-Leu-Ala-Ala-Ile-Leu-Gly-Arg-Arg-NH₂
Peptide 4 (SEQ ID No. 20):
Acetyl-His-Ser-Asp-Ala-Val-Phe-Thr-Asp-Asn-Tyr-Thr-Arg-Leu-Arg-Arg-Gln-Leu-Ala-Val-Arg-Arg-Tyr-Leu-Ala-Ala-Ile-Leu-Gly-Arg-Arg-NH₂
Peptide 5 (SEQ ID No. 21):
Stearoyl-His-Ser-Asp-Ala-Val-Phe-Thr-Asp-Asn-Tyr-Thr-Arg-Lue-Arg-Arg-Gln-Leu-Ala-Val-Arg-Arg-Tyr-Leu-Ala-Ala-Ile-Leu-Gly-Arg-Arg-NH₂
Peptide 6 (SEQ ID No. 22):
His-Ser-Asp-Ala-Val-Phe-Thr-Asp-Asn-Tyr-Thr-Arg-Leu-Arg-Arg-Gln-Leu-Ala-Val-Arg-Arg-Tyr-Leu-Ala-Ala-Ile-Leu-Gly-Arg-Arg-OH
Peptide 7 (SEQ ID No. 23):
His-Ser-Asp-Ala-Val-Phe-Thr-Asp-Asn-Tyr-Thr-Arg-Leu-Arg-Arg-Gln-Nle-Ala-Val-Arg-Arg-Tyr-Leu-Ala-Ala-Ile-Leu-Gly-Arg-Arg-NH₂
Peptide 8 (SEQ ID No. 24):
His-Ser-Asp-Ala-Val-Phe-Thr-Asp-Asn-Tyr-Thr-Arg-Leu-Arg-Arg-Gln-Leu-Ala-Val-Arg-Arg-Tyr-Leu-Ala-Ala-Ile-Leu-Gly-NH₂
Peptide 9 (SEQ ID No. 25):
His-Ser-Asp-Ala-Val-Phe-Thr-Asp-Asn-Tyr-Thr-Arg-Leu-Arg-Arg-Gln-Leu-Ala-Val-Arg-Arg-Tyr-Leu-Ala-Ala-Ile-Leu-Gly-Lys-NH₂
Peptide 10 (SEQ ID No. 26):
His-Ser-Asp-Ala-Val-Phe-Thr-Asp-Asn-Tyr-Thr-Arg-Leu-Arg-Arg-Gln-Leu-Ala-Val-Arg-Arg-Tyr-Leu-Ala-Ala-Ile-Leu-Gy-Arg-NH₂
Peptide 11 (SEQ ID No. 27):
His-Ser-Asp-Ala-Val-Phe-Thr-Asp-Asn-Tyr-Thr-Arg-Leu-Arg-Arg-Gln-Leu-Ala-Val-Arg-Arg-Tyr-Leu-Ala-Ala-Ile-Leu-Gly-Lys-Arg-NH₂
Peptide 12 (SEQ ID No. 28):
His-Ser-Asp-Ala-Val-Phe-Thr-Glu-Asn-Tyr-Thr-Arg-Leu-Arg-Arg-Gln-Leu-Ala-Val-Arg-Arg-Tyr-Leu-Ala-Ala-Ile-Leu-Gly-Arg-Arg-NH₂
Peptide 13 (SEQ ID No. 29):
His-Ser-Asp-Ala-Val-Phe-Thr-Glu-Asn-Tyr-Thr-Arg-Lue-Arg-Arg-Gln-Leu-Ala-Val-Arg-Arg-Tyr-Leu-NH₂
Peptide 14 (SEQ ID No. 30):
His-Ser-Asp-Ala-Val-Phe-Thr-Ala-Asn-Tyr-Thr-Arg-Leu-Arg-Arg-Gln-Leu-Ala-Val-Arg-Arg-Tyr-Leu-Ala-Ala-Ile-Leu-Gly-Arg-Arg-NH₂
Peptide 15 (SEQ ID No. 31):
His-Ser-Asp-Ala-Val-Phe-Thr-Ala-Asn-Tyr-Thr-Arg-Leu-Arg-Arg-Gln-Leu-Ala-Vla-Arg-Arg-Tyr-Leu-NH₂
Peptide 16 (SEQ ID No. 32):
His-Ser-Asp-Ala-Val-Phe-Tyr-Asp-Asn-Tyr-Thr-Arg-Leu-Arg-Arg-Gln-Leu-Ala-Val-Arg-Arg-Tyr-Leu-Ala-Ala-Val-Leu-Gly-Arg-Arg-NH₂
Peptide 17 (SEQ ID No. 33):
His-Ser-Asp-Ala-Ile-Phe-Thr-Asp-Ser-Tyr-Ser-Arg-Tyr-Arg-Arg-Gln-Leu-Ala-Val-Arg-Arg-Tyr-Leu-Ala-Ala-Val-Leu-Gly-Arg-Arg-OH
Peptide 18 (SEQ ID No. 34):
His-Ser-Asp-Ala-Ile-Phe-Thr-Asp-Ser-Tyr-Ser-Arg-Tyr-Arg-Arg-Gln-Leu-Ala-Val-Arg-Arg-Tyr-Leu-Ala-Ala-Ile-Leu-Gly-Arg-Arg-NH₂
Peptide 19 (SEQ ID No. 35):
Acetyl-His-Ser-Asp-Ala-Ile-Phe-Thr-Asp-Ser-Tyr-Ser-Arg-Tyr-Arg-Arg-Gln-Leu-Ala-Val-Arg-Arg-Tyr-Leu-Ala-Ala-Val-Leu-Gly-Arg-Arg-NH₂
Peptide 20 (SEQ ID No. 36):
His-Ser-Asp-Ala-Ile-Phe-Thr-Asp-Ser-Tyr-Ser-Arg-Tyr-Arg-Arg-Gln-Leu-Ala-Val-Arg-Arg-Tyr-Leu-Ala-Ala-Val-Leu-Gly-Arg-Arg-Tyr-Arg-Gln-Arg-Val-Arg-Asn-Arg-NH₂
Peptide 21 (SEQ ID No. 37):
His-Ser-Asp-Ala-Ile-Phe-Thr-Asp-Ser-Tyr-Ser-Arg-Tyr-Arg-Arg-Gln-Leu-Ala-Val-Arg-Arg-Tyr-Leu-Ala-Ala-Ile-Leu-Gly-Arg-Arg-Tyr-Arg-Gln-Arg-Val-Arg-Asn-Arg-NH₂
Peptide 22 (SEQ ID No. 38):
His-Ser-Asp-Ala-Ile-Phe-Thr-Asp-Ser-Tyr-Ser-Arg-Tyr-Arg-Arg-Gln-Leu-Ala-Val-Arg-Arg-Tyr-Leu-NH₂
Peptide 23 (SEQ ID No. 39):
Acetyl-His-Ser-Asp-Ala-Ile-Phe-Thr-Asp-Ser-Tyr-Ser-Arg-Tyr-Arg-Arg-Gln-Leu-Ala-Val-Arg-Arg-Tyr-Leu-NH₂
Peptide 24(SEQ ID No. 40):
His-Ser-Asp-Ala-Ile-Phe-Thr-Asp-Ser-Tyr-Ser-Arg-Tyr-Arg-Arg-Gln-Leu-Ala-Val-Arg-Arg-Tyr-Leu-Ala-Ala-Val-Leu-Gly-Arg-Arg-NH₂

### Test Example 1

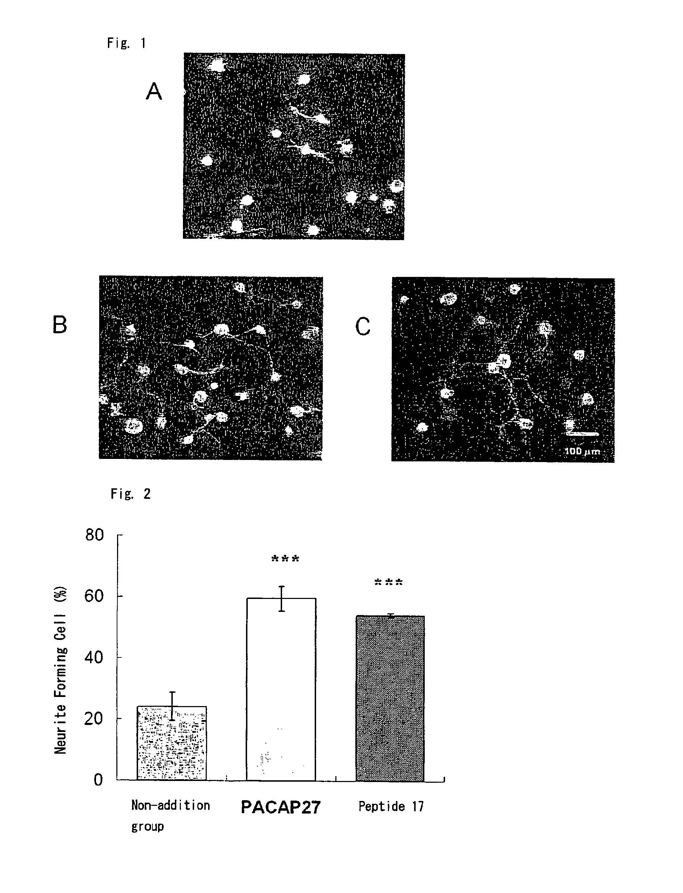

Promoting effect on neuritogenesis in cultured rabbit trigeminal nerve cells (1)
1. Animals Used
   Japanese white rabbits (2 to 3 days old) purchased from Kitayama Labes Co., Ltd. were used.
2. Test Substance
   PACAP27 and peptide 17
3. Test Method
   (1) Cell Cultivation
      Rabbit trigeminal nerve cells were isolated according to a method reported by Chan et al. (Kwan Y. Chan and Richard H. Haschke, Exp. Eye Res. 41: 687-699, 1985). The rabbit was subjected to cardiac perfusion with physiological saline under ether anesthesia. A trigeminal ganglion was excised from the rabbit, and then dispersed in a nerve dispersion liquid (Sumitomo Bakelite Co., Ltd.) to prepare trigeminal nerve cells. The trigeminal nerve cells were cultured in Neurobasal medium (Invitrogen Corp) supplemented with B27 Supplement (Invitrogen Corp; final concentration: 2% v/v) and L-glutamine (Invitrogen Corp; final concentration: 1 mM) under 5% CO₂ and 95% air at a humidity of 100% and a temperature of 37°C. The cells were seeded on a polylysine/laminine coated cover glass (Sumitomo Bakelite Co., Ltd.) put in a 24-well plate at about 3 × 10³ cells/well. PACAP27 (final concentration: 1 µM) or peptide 17 (final concentration: 1 µM) was added to the culture medium, and as a control, neither of them were added to the culture medium (a non-addition group).
   (2) Staining
      After cultured for 48 hours, the rabbit trigeminal nerve cells were fixed with 4% paraformaldehyde at room temperature for 2 hours. An anti-neurofilament 200 antibody (Sigma) that specifically recognizes neurofilaments constituting nerve cell bodies and neurites, and a fluorescent secondary antibody (Molecular Probes, Inc.) that reacts to the anti-neurofilament 200 antibody were used to fluorescently stain the fixed sample. The stained cells were observed with a fluorescence microscope, and cell images were scanned into a computer.
   (3) Image analysis
      For evaluating neuritogenesis in the cultured rabbit trigeminal nerve cells, the lengths of the longest neurite and the major axis of the cell body in the stained cell images scanned into the computer were measured using image analysis software (Image-Pro Plus ver. 4.0, Planetron, Inc.) The cells in 3 wells for each group were measured. A cell having a neurite of which length is two or more times longer than the length of the major axis of the cell body was defined as a neurite forming cell. The proportion (%) of the neurite forming cells in the total cell number was calculated (Otori, Y., Wei, J.Y., Barnstable, C. J., Invest. Ophthalmol. Vis. Sic., (1998)39, 972-981).
4. Test Results
   Fig. 1 shows fluorescence microscope images of the cultured rabbit trigeminal nerve cells, and (A) non-addition group, (B) PACAP27-addition group and (C) peptide 17-addition group are shown. In the non-addition group, few neurite forming cells having elongated neurites stained by fluorescent immunostaining were observed. In the PACAP27-addition group and the peptide 17-addition group, a larger number of neurite forming cells were observed.
   Fig. 2 is a graph showing the proportion (%) of the neurite forming cells in the total cell number in each addition group. The proportions (%) of the neurite forming cells were 24.1% in the control group, 59.5% in the PACAP27-addition group and 54.1% in the peptide 17-addition group. As a result of statistical analysis, significant promoting effects on neuritogenesis were shown in the PACAP27-addition group and the peptide 17-addition group as compared with the non-addition group (N = 3, average value ± standard deviation, *** : p<0.001 vs non-addition group, Dunnett's multiple comparison analysis).
   As evident from the above-described results, PACAP27 and peptide 17 have a promoting effect on neuritogenesis in the cultured rabbit trigeminal nerve cells.

### Test Example 2

### Improving effect on reduced sensitivity of the rabbit cornea caused by using a microkeratome (1)

1. Animal Used
Japanese white rabbits with a body weight of 1.5 to 2.0 kg purchased from Kitayama Labes Co., Ltd were used. The animals were each put in separate cages placed in a room wherein a temperature of 23 ± 3°C and a humidity of 55 ± 10% were kept and a light was on for 12 hours per day (lighting at 8:00 and lights-out at 20:00), and raised after arrival until the final day of the test. The animals were given 100g of solid feed (Labo R Stock, Nosan Corporation) per day, and could freely have tap water.
2. Test Substance
PACAP27 was used as a test substance. The test substance was dissolved in the following vehicle solution at 10 µM (about 0.003%) to prepare eye drops. As a control, the following vehicle solution that does not contain the test substance was used.

| Formulation of a vehicle solution: | |
|---|---|
| sodium dihydrogen phosphate dihydrate | 0.1g |
| sodium chloride | 0.9g |
| sodium hydroxide | appropriate amount |
| purified water | appropriate amount |
| | 100 mL(pH 7.0) |

3. Test Method
(1) Grouping
   On the day before administration of the eye drops, the anterior eye of the animal was observed by macroscopy, while the corneal spots stained with fluorescein were observed. Rabbits confirmed that they had no abnormality by the observations were selected. Before starting to administer the eye drops, the initial values of corneal sensitivity of the selected rabbits were measured using the Cochet-Bonnet aesthesiometer (Luneau). The rabbits were divided into groups by a multivariable blocking allocation method using SAS preclinical package (version 5.0, SAS institute Japan) so that the initial corneal sensitivity values in all groups were equal.
(2) Creation of corneal flap
   The animal was systemically anesthetized by intramuscular injection (0.9 mL/kg) of a mixture of Ceractal (2% xylazine) and Ketaral (5% ketamine) in a ratio of 0.5:1. After sufficient exposure of the eyeballs, an adaptor for rabbit eyes was attached to the inside of a suction ring. A microkeratome (MK2000, Nidek Co., Ltd.) (Arbelaez, M. C. et al., J. Refract. Surg. 2002, May-jun; 18 (3 Suppl) : S357-60) with a blade having a thickness of 130 µm was used to create a corneal flap with a diameter of 8.5 mm. The corneal flap was precisely returned to the original position under a microscope, and the animal was made to awake from anesthesia while the animal was watched so that the flap did not move.
(3) Administration
   The eye drops containing the test substance, or the vehicle solution was administered by ocular instillation for 8 weeks from the day of creation of the corneal flap. The eye drops or the vehicle solution was administered to the operated eye 4 times a day at 2-hour intervals in an amount of 50 µL per time using a micro-pipette. For 1 week after the surgery, 0.3% ofloxacin eye drops (Tarivid eye drop, Santen Pharmaceutical Co., Ltd.) were administered by ocular instillation 4 times a day simultaneously with administration of the test eye drops.
(4) Measurement of corneal sensitivity
   Corneal sensitivity was measured once a week from the first week to the eighth week after the surgery using the Cochet-Bonnet aesthesiometer. The measurement was blind trial so that an examiner could not know which group the rabbit being measured belonged to.
(5) Statistical analysis
   For statistical analysis, a two group t-test (SAS preclinical package; version 5.0, SAS Institute Japan) was used, and the significance level was defined as less than 5%.

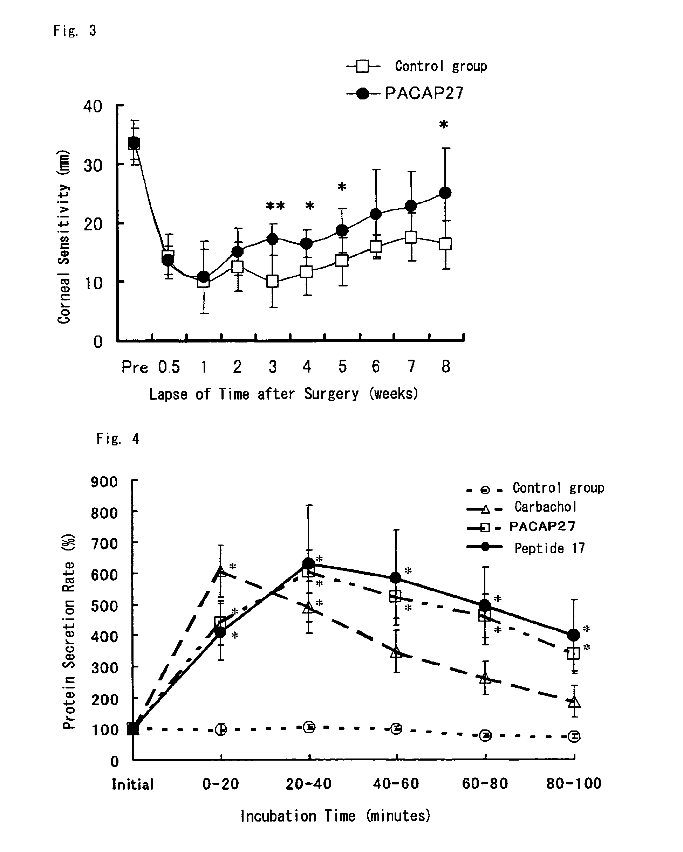

4. Test Results
Fig. 3 shows time-dependent changes in corneal sensitivity. Corneal sensitivity is indicated by the longest length of a nylon filament which induces an eyeblink response when the tip of the nylon filament (diameter: 0.12 mm) attached to the Cochet-Bonnet aesthesiometer is contacted the center of a cornea, and the longest length is shown as the vertical axis in Fig. 3. On the first week after the surgery, sharp declines in corneal sensitivity were found in the vehicle solution-administration group and the PACAP27-administration group. After that, slow recoveries of corneal sensitivity were found in the vehicle solution-administration group and the PACAP27-administration group. When comparing the corneal sensitivity in the PACAP27-administration group was compared with the corneal sensitivity in the vehicle solution-administration group at each measuring point, the corneal sensitivity in the PACAP27-administration group was significantly high on 3, 4, 5 and 8th weeks from the start of administration (n = 6 to 7, average value ± standard deviation, *p<0.05, **p<0.005, t-test).

In laser assisted in situ keratomileusis (LASIK), a corneal flap (cornea piece) created by using a microkeratome is folded to expose a corneal stroma, and the corneal stroma is abraded by excimer laser irradiation to correct the refractive error. Then, the corneal flap is returned to the original position. In this test example, the effect of the pharmaceutical composition of the present invention on a rabbit model having reduced corneal sensitivity was examined using a microkeratome. As evident from the above-described results, PACAP27 can promote the recovery of reduced corneal sensitivity.

### Test Example 3

### Promoting effect on protein secretion from an excised lacrimal gland (1)

A lacrimal gland was excised from a rabbit and then treated with PACAP27 or peptide 17. Pharmacological effects of PACAP27 and peptide 17 on an increase in the protein secretion amount were examined.
1. Preparation of test substance solution
   (a) Purified water was added to sodium chloride (137.92 g), potassium chloride (7.01 g), calcium chloride dihydrate (3.53 g) and magnesium chloride hexahydrate (1.2 g) so that the total volume was 1000 mL (solution 1). Purified water was added to potassium dihydrogen phosphate (8.17 g) so that the total volume was 500 mL (solution 2). The solution 1 (50 mL) and the solution 2 (10 mL) were added to 900 mL of purified water, and in the resulting solution were dissolved glucose (2.07 g) and sodium hydrogen carbonate (2.1 g). The total volume of the resulting solution was adjusted to 1000 mL (pH about 7.4), which was used as a culture solution. A mixed gas of 95% oxygen and 5% carbon dioxide was allowed to pass through the culture solution.
   (b) PACAP27 or peptide 17 was dissolved in the culture solution at a concentration of 10⁻⁷ M, which was used as a test substance solution. Carbachol (Nacalai Tesque, Inc.), which has muscarinic agonist activity, was dissolved in the culture solution at a concentration of 10⁻⁴ M, which was used as a control of the drugs (the carbachol-addition group). The culture solution to which no drug was added was used as a control group.
2. Preparation of a lacrimal gland section sample
   A male Japanese white rabbit was subjected to general anesthesia, and perfused with a perfusion solution (containing 116 mM sodium chloride and 5.4 mM potassium chloride) through the abdominal aorta. Main lacrimal gland tissue was excised from the rabbit. After adipose connective tissue was removed from the excised main lacrimal gland tissue, the main lacrimal gland tissue was divided into two equal sections (about 40 mg per section). The lacrimal gland sections were transferred to a 24 well plate filled with 0.5 mL/well of the culture solution, and then incubated at 37°C. The culture solution was replaced by a fresh culture solution three times every 20 minutes. The incubation was carried out 60 minutes in total to obtain lacrimal gland section samples in a stationary state.
3. Method of experiment
   (a) The section sample in a stationary state was incubated at 37°C for 20 minutes in 0.5 mL of a fresh culture solution. The culture solution was then collected. The amount of protein in the culture solution was determined by using a protein staining reagent (DC Protein Assay Reagent, Bio Rad), and was converted into the protein secretion amount per 1 mg of the wet sample. The protein secretion amount thus obtained was assumed to be a protein secretion rate of 100%, which was regarded as the secretion rate before treatment.
   (b) The culture solution of the section sample after incubation obtained in (a) was replaced by 0.5 mL of the test substance solution. The test substance solution was replaced by a fresh test substance solution and then collected five times every 20 minutes. The protein amount in the collected solution was determined by using the protein staining reagent. In the same way, as a control group, the section sample was incubated in the culture solution which was replaced by a fresh culture solution and the protein amount in the culture solution was determined.
4. Results

The results of the experiments are shown in Fig. 4. The vertical axis shows the secretion rate (n= 6, average value ± standard deviation) of the rabbit excised lacrimal gland when the rate before the treatment is assumed to be 100%. The horizontal axis shows lapse time. In the carbachol-addition group, a significant increase in the protein secretion rate with a peak at the time from 0 to 20 minutes was shown. In the group treated with 10⁻⁷ M PACAP27 or peptide 17, the protein secretion rate was significantly increased at 20 minutes after initiation of the treatment and thereafter, and then reached the maximum at between 40 and 60 minutes after initiation of the treatment (*; p<0.05, Dunnett's multiple comparison analysis). The action of the drug lasted longer in the PACAP27 or peptide 17-addition group, as compared with the carbachol-addition group. In the control group, such an increase in the protein secretion rate was not observed.

As evident from the above-described results, PACAP27 and peptide 17 have an excellent promoting effect on protein secretion in an excised lacrimal gland. In other words, PACAP27 and peptide 17 have a promoting effect on secretion of tear and are useful for treating a dry eye symptom.

### Test Example 4

### Promoting effect on neuritogenesis in cultured rabbit trigeminal nerve cells (2)

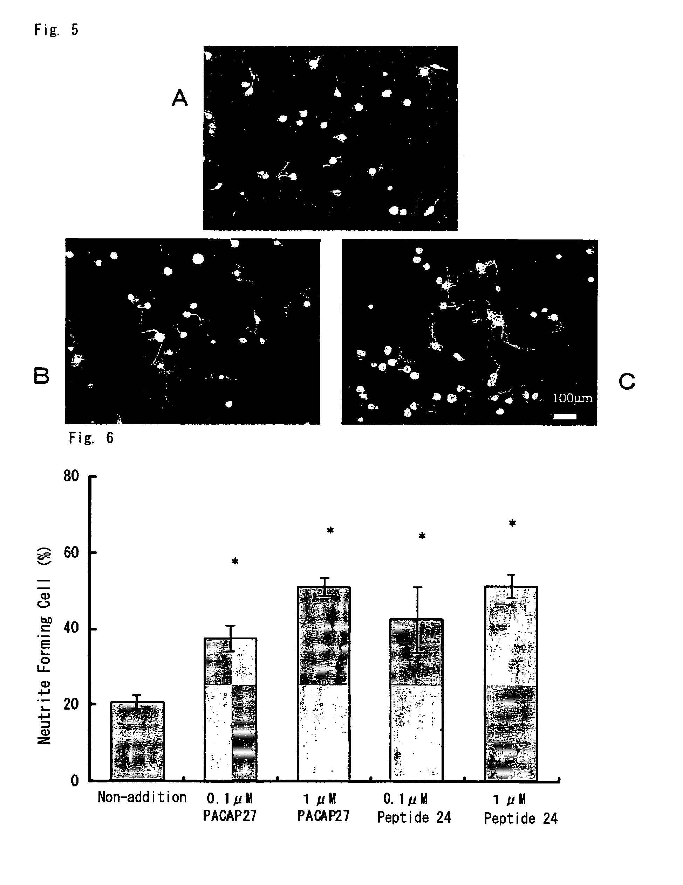

1. Used animal
   Japanese white rabbits (2 to 3 days old) purchased from Kitayama Labes Co., Lid. were used.
2. Test substance
   PACAP27 and peptide 24
3. Test method
   (1) Cell culture
      Rabbit trigeminal nerve cells were isolated according to a method reported by Chan et al. (Kwan Y. Chan and Richard H. Haschke, Exp. Eye Res. 41: 687-699, 1985). The rabbit was subjected to cardiac perfusion with physiological saline under ether anesthesia. A trigeminal ganglion was excised from the rabbit, and then dispersed in a nerve dispersion liquid (Sumitomo Bakelite Co., Ltd.) to prepare trigeminal nerve cells. The trigeminal nerve cells were cultured in Neurobasal medium (Invitrogen Corp) supplemented with B27 Supplement (Invitrogen Corp; final concentration: 2% v/v) and L-glutamine (Invitrogen Corp; final concentration: 1 mM) under 5% CO₂ and 95% air at a humidity of 100% and a temperature of 37°C. The cells were seeded on a polylysine/laminine coated cover glass (Sumitomo Bakelite Co., Ltd.) put in a 24-well plate at about 3 × 10³ cells/well. PACAP27 (final concentration: 0.1 µM, 1 µM) or peptide 24 (final concentration: 0.1 µM, 1 µM) was added to the culture medium, and as a control, neither of them were added to the culture medium (a non-addition group).
   (2) Staining
      After cultured for 48 hours, the rabbit trigeminal nerve cells were fixed with 4% paraformaldehyde at room temperature for 2 hours. An anti-neurofilament 200 antibody (Sigma) that specifically recognizes neurofilaments constituting nerve cell bodies and neurites, and a fluorescent secondary antibody (Molecular Probes, Inc.) that reacts to the anti-neurofilament 200 antibody were used to fluorescently stain the fixed sample. The stained cells were observed with a fluorescence microscope, and cell images were scanned into a computer.
   (3) Image analysis
      For evaluating neuritogenesis in the cultured rabbit trigeminal nerve cells, the length of the longest neurite and the major axis of the cell body in the stained cell images scanned into the computer were measured using image analysis software (Image-Pro Plus ver. 4.0, Planetron, Inc.) The cells in 3 wells for each group were measured. A cell having a neurite of which length is two or more times longer than the length of the major axis of the cell body was defined as a neurite forming cell. The proportion (%) of the neurite forming cells in the total cell number was calculated (Otori, Y., Wei, J.Y., Barnstable, C. J., Invest. Ophthalmol. Vis. Sic., (1998)39, 972-981).
   (4) Statistical Analysis
      For statistical analysis, a Dunnett's multiple comparison analysis method (SAS preclinical package, version 5.4, SAS Institute Japan) was used, and the significance level was defined as less than 5%.
4. Test results
   Fig. 5 shows fluorescence microscope images of the cultured rabbit trigeminal nerve cells. (A) non-addition group, (B) PACAP27-addition group and (C) peptide 24-addition group are shown. In the non-addition group, the number of neurite forming cells having elongated neurites was small. In the PACAP27-addition group and the peptide 24-addition group, many neurite forming cells were observed.

Fig. 6 is a graph showing the proportion (%) of the neurite forming cells in the total cell number in each addition group. The proportions (%) of the neurite forming cells were 20.7% in the control group, 37.6% and 51.1% in the 0.1 µM and 1 µM PACAP27-addition groups respectively, and 42.5% and 51.4% in the 0.1 µM and 1 µM peptide 24-addition groups respectively. As a result of statistical analysis, significantly increased neurite forming cells were found in the PACAP27- and peptide 24-addition groups, as compared with the non-addition group.

As evident from the above-described results, PACAP27 and peptide 24 have a promoting effect on neuritogenesis in the cultured rabbit trigeminal nerve cells.

### Test Example 5

### Promoting effect on protein secretion from an excised lacrimal gland (2)

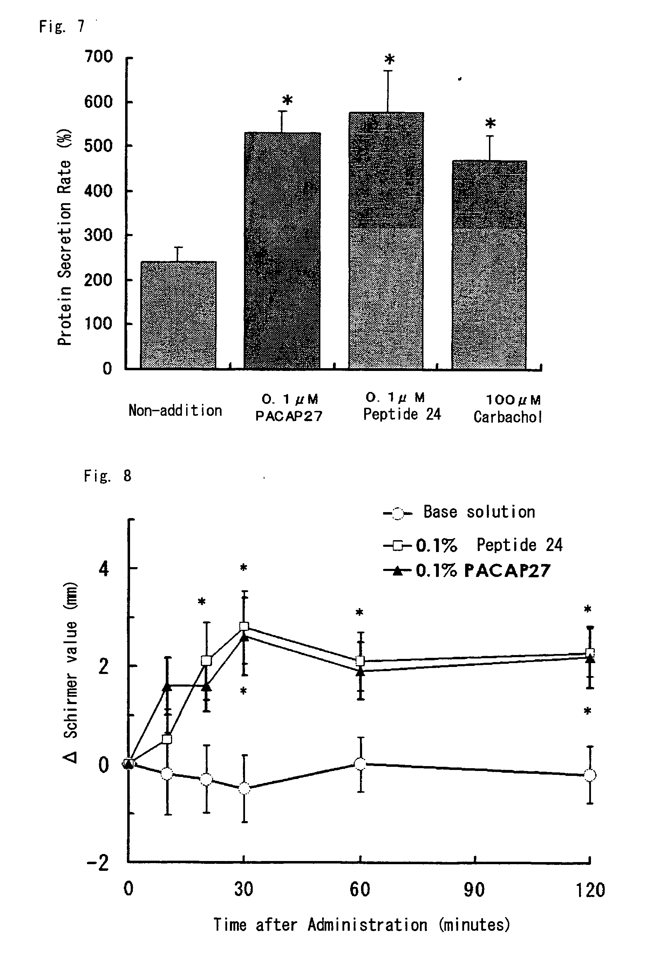

A lacrimal gland was excised from a rabbit and incubated in a solution containing PACAP27 or peptide 24, and changes in the amount of secreted protein were examined as lacrimal gland stimulating activity.
1. Used animal
   Japanese white rabbits with a body weight of 1.5 to 2.0 kg purchased from Fukusaki Rabbit warren were used. The animals were each put in separate cages placed in a room wherein a temperature of 23 ± 3°C and a humidity of 55 ± 10% were kept and a light was on for 12 hours per day (lighting at 8:00 and lights-out at 20:00), and raised after arrival until the final day of the test. The animals were given 100g of solid feed (Labo R Stock, Nosan Corporation) per day, and could freely have tap water.
2. Test substance
   PACAP27 and peptide 24 were used as test substances. Test substance solutions were prepared as follows.
   (1) Purified water was added to sodium chloride (137.92 g), potassium chloride (7.01 g), calcium chloride dihydrate (3.53 g) and magnesium chloride hexahydrate (1.2 g) so that the total volume was 1000 mL (solution 1). Purified water was added to potassium dihydrogen phosphate (8.24 g) so that the total volume was 500 mL (solution 2). The solution 1 (50 mL) and the solution 2 (10 mL) were added to 900 mL of purified water, and in the resulting solution were dissolved glucose (2.07 g) and sodium hydrogen carbonate (2.1 g). The total volume of the resulting solution was adjusted to 1000 mL (pH about 7.4), which was used as an incubation solution.
   (2) PACAP27 or peptide 24 was dissolved in the incubation solution at a concentration of 10⁻⁷ M, which was used as a test substance solution. Carbachol (Nacalai Tesque, Inc.), which has muscarinic agonist activity, was dissolved in the incubation solution at a concentration of 10⁻⁴ M, which was used as a control of the drugs. The incubation solution to which no drug was added was used as a control group. A mixed gas of 95% oxygen and 5% carbon dioxide was allowed to pass through each incubate solution in advance to the test.
3. Test method
   (1) Preparation of a lacrimal gland section sample
      The animal was subjected to general anesthesia, and perfused with a perfusion solution (containing 116 mM sodium chloride and 5.4 mM potassium chloride) through the abdominal aorta. Main lacrimal gland tissue was excised from the animal. After adipose connective tissue was removed from the excised main lacrimal gland tissue, the main lacrimal gland tissue was divided into two equal sections (about 40 mg per section). The lacrimal gland sections were transferred to a 24 well plate filled with 0.5 mL/well of the incubation solution, and then incubated at 37°C under a mixed gas atmosphere of 95% oxygen and 5% carbon dioxide. The incubation solution was replaced by a fresh incubation solution three times every 20 minutes. The incubation was carried out 60 minutes in total to obtain lacrimal gland section samples in a stationary state.
   (2) Quantification of secreted protein
      The section sample in a stationary state was incubated in 0.5 mL of a fresh incubation solution at 37°C for 20 minutes under a mixed gas atmosphere of 95% oxygen and 5% carbon dioxide. After completion of the incubation, the incubation solution was collected, and the protein amount in the solution was determined by using a protein staining reagent (DC Protein Assay Reagent, Bio Rad). The protein amount thus obtained was regarded as the amount of the protein secreted from the excised lacrimal gland section sample before initiation of the test. Subsequently, the incubation solution was replaced by 0.5 mL of an incubation solution containing the test substance, and the section sample was incubated at 37°C for 60 minutes under a mixed gas atmosphere of 95% oxygen and 5% carbon dioxide. The incubation solution was collected, and the protein amount in the incubation solution was determined. For the control group and the carbachol group, in the same way as described above, the incubation solutions were replaced by respective incubation solutions, and the section samples were incubated 37°C for 60 minutes under a mixed gas atmosphere of 95% oxygen and 5% carbon dioxide, and the protein amounts in the incubation solutions were determined. The protein amount in the incubation solution was converted into the protein amount per 1 mg of wet weight of the lacrimal gland section sample. The protein secretion rate before initiation of the test was assumed to be 100%. The protein secretion rate after incubation in the incubation solution containing the test substance was calculated, and a promoting effect of the test substance on protein secretion was examined.
   (3) Statistical analysis
      For statistical analysis, a Dunnett's multiple comparison analysis method (SAS preclinical package, version 5.4, SAS Institute Japan) was used, and the significance level was defined as less than 5%.
4. Test results
   The results of the test are shown in Fig. 7. The vertical axis shows the protein secretion rate of the excised rabbit lacrimal gland when the rate before the treatment is assumed to be 100%. In the carbachol group, the PACAP27 group and the peptide 24 group, a significant increase in the protein secretion rate was found, as compared with the control group containing no drug (*p<0.05).
   As evident from the above-described results, PACAP27 and peptide 24 have a promoting effect on protein secretion from the excised lacrimal gland.

### Test Example 6

### Promoting effect on tear secretion in a rabbit

Changes in the tear amount when PACAP27 and peptide 24 were administered to the eyes of a rabbit were examined.
1. Used animal
Japanese white rabbits with a body weight of 1.5 to 2.0 kg purchased from Kitayama Labes Co., Ltd were used. The animals were each put in separate cages placed in a room wherein a temperature of 23 ± 3°C and a humidity of 55 ± 10% were kept and a light was on for 12 hours per day (lighting at 8:00 and lights-out at 20:00), and raised after arrival until the final day of the test. The animals were given 100g of solid feed (Labo R Stock, Nosan Corporation) per day, and could freely have tap water.
2. Test Substance
PACAP27 and peptide 24 were used as test substances. Each test substance was dissolved in the following vehicle solution at a concentration of 0.1% to prepare eye drops. As a control, the following vehicle solution was used.

| Formulation of a vehicle solution: | |
|---|---|
| sodium dihydrogen phosphate dihydrate | 0.1g |
| sodium chloride | 0.9g |
| sodium hydroxide | appropriate amount |
| purified water | appropriate amount |
| | 100 mL(pH 7.0) |

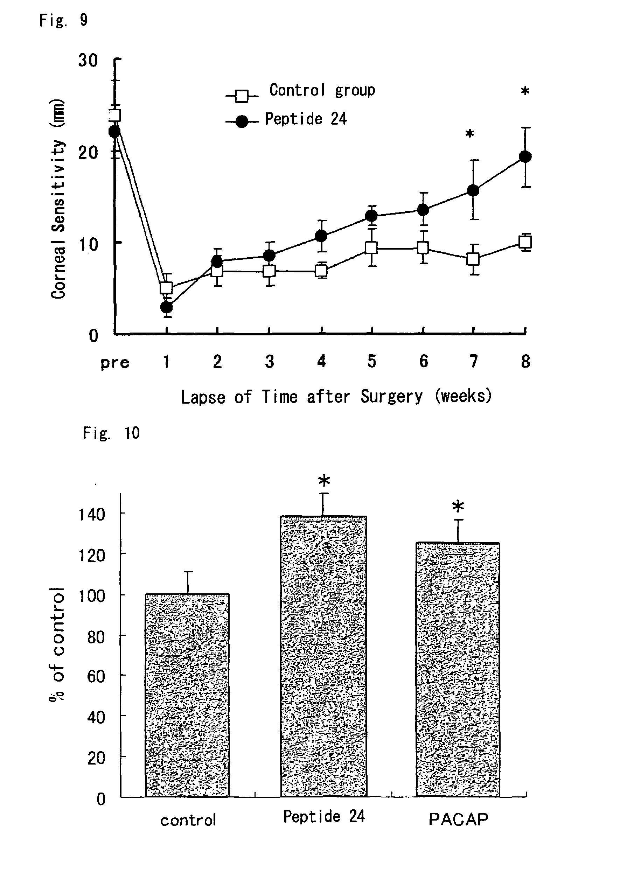

3. Test method
The effect of the eye drops of the test substance or the vehicle solution on tear secretion was examined by measuring the amount of tear secreted, when 50 µL of the eye drops containing the test substance, or the vehicle solution was administered to one eye of a rabbit. The amount of tear secreted was measured at 8.5 minutes before administration, and at 10 minutes, 20 minutes, 30 minutes, 60 minutes and 120 minutes after the administration by a Schirmer method. The rabbit was locally anesthetized by administering 10 µL of 0.4% oxybuprocaine hydrochloride (Benoxil eye drops, Santen Pharmaceutical Co., Ltd.) to the eye 7.5 minutes before measurement of the tear amount. After the tear in the lower eyelid conjunctival sac was wiped with filter paper, the amount of tear secreted in 1 minute was measured using a Schirmer's test strip (Showa Yakuhin Kako Co.). The difference between the tear secretion amount at each time point after administration of the eye drops and the tear secretion amount before administration of the eye drops was calculated as a change in the tear amount caused by the test substance.
4. Statistical analysis
The changes in the tear amount were compared between the vehicle solution-administration group and the drug-administration group by using a Dunnett's multiple comparison analysis method (SAS preclinical package, Version 5.4, SAS Institute Japan), and the significance level was defined as less than 5%.
5. Test results
Fig. 8 shows changes in the amount of tear secreted when eye drops were administered to the eye of a rabbit. In the vehicle solution-administration group, no significant change was observed from immediately after administration to 120 minutes after administration. A significant increase in the tear secretion amount was observed 30 minutes and 120 minutes after administration in the PACAP27-administration group and from 20 minutes to 120 minutes after administration in the peptide 24-administration group, as compared with the vehicle solution-administration group.
As evident from the above-described results, PACAP27 and peptide 24 have a promoting effect on tear secretion, and may be useful for ameliorating a tear-deficient type dry eye symptom.

### Test Example 7

### Improving effect on reduced sensitivity of the rabbit cornea caused by using a microkeratome (2)

1. Used animal
Male Japanese white rabbits with a body weight of 1.5 to 2.0 kg purchased from Kitayama Labes Co., Ltd were used. The animals were each put in separate cages placed in a room wherein a temperature of 23 ± 3°C and a humidity of 55 ± 10% were kept and a light was on for 12 hours per day (lighting at 8:00 and lights-out at 20:00), and raised after arrival until the final day of the test. The animals were given 100g of solid feed (Labo R Stock, Nosan Corporation) per day, and could freely have tap water.
2. Test Substance
Peptide 24 was used as a test substance. The test substance was dissolved in the following vehicle solution at a concentration of 0.01% to prepare eye drops. As a control, the following vehicle solution that does not the test substance was used.

| Formulation of a vehicle solution: | |
|---|---|
| sodium dihydrogen phosphate dihydrate | 0,1g |
| sodium chloride | 0.9g |
| sodium hydroxide | appropriate amount |
| purified water | appropriate amount |
| Total volume | 100 mL(pH 7.0) |

3. Test method
(1) Creation of corneal flap
   The animal was systemically anesthetized by intramuscular injection (0.9 mL/kg) of a mixture of Ceractal (2% xylazine) and Ketaral (5% ketamine) in a ratio of 0.5:1. After sufficient exposure of the eyeballs, a microkeratome (Nidek Co., Ltd.) was used to create a corneal flap with a diameter of 8.5 mm. For this surgery, an adapter for a rabbit and a blade with a thickness of 130 µm were used.
(2) Administration by ocular instillation
   The eye drops containing the test substance, or the vehicle solution was administered continuously by ocular instillation for 8 weeks from the day of creation of the corneal flap. The eye drops or the vehicle solution was administered 4 times a day at 2-hour intervals in an amount of 50 µL per time. For 1 week from the day of creation of the corneal flap, one drop of Niflan eye drops (Senju Pharmaceutical Co., Ltd.) as an anti-inflammatory agent was administered simultaneously with the first and third administration per day of the test substance or the vehicle solution, and one drop of 0.3% gatifloxacin eye drops (Gatiflo eye drops, Senju Pharmaceutical Co., Ltd.) was administered by ocular instillation simultaneously with the second and fourth administration per day of the test substance or the vehicle solution.
(3) Measurement of corneal sensitivity
   Corneal sensitivity was measured before the surgery and once a week from the first week to the eighth week after the surgery using the Cochet-Bonnet aesthesiometer.
   Corneal sensitivity was determined by the frequency of eyeblink responses induced when a nylon filament attached to the Cochet-Bonnet aesthesiometer was contacted the center of the cornea of the animal. When five times or more of eyeblink responses were confirmed for 10 times of touch of the aesthesiometer filament, the animal was judged to have corneal sensitivity. When the frequency of eyeblink responses was less than 5 times for 10 times of touch of the aesthesiometer filament, the animal was judged to have no corneal sensitivity. This determination was repeated three times. When the animal was judged to have corneal sensitivity twice or more, the maximum value (the longest length of the nylon filament of the aesthesiometer) was recorded as corneal sensitivity of the individual at that measurement time point. The measurement was blind trial so that an examiner could not know which group the rabbit being measured belonged to.

4. Statistical analysis
Corneal sensitivity at each measurement time point was compared between the vehicle solution-administration group and the test substance-administration group by a t-test (SAS preclinical package, Version 5.0, SAS Institute Japan), and the significance level was defined as less than 5%.
5. Test result
Fig. 9 shows reduction in corneal sensitivity caused by cutting the corneal nerve, and changes of corneal sensitivity thereafter. In both the control group and the peptide 24-administration group, rapidly reduced corneal sensitivity was observed 1 week after creation of the corneal flap. After that, almost no recovery of corneal sensitivity was seen in the control group, while corneal sensitivity was slowly recovered in the peptide 24-administration group. A statistically significant improving effect on corneal sensitivity was observed in the peptide 24-administration group 7 and 8 weeks after creation of the corneal flap, as compared with the control group. As evident from the above-described results, peptide 24 has an improving effect on corneal sensitivity.

### Test Example 8

### Promoting effect on proliferation of cultured corneal epithelial cells

1. Used cell
   Human normal corneal epithelial cells (Kurabo Industries Ltd., strain No. 4C0466) were used.
2. Test substance and Preparation method
   Peptide 24 and PACAP27 were used as test substances. The test substance was dissolved in distilled water at a concentration 1000 times of the final concentration (10⁻⁵ M), and the concentration was then adjusted to twice the final concentration with a serum-free proliferation medium for human corneal epithelial cells (EpiLife (-), Kurabo Industries Ltd.).
3. Test method
   (1) Cultivation and Addition of test substance
      After thawing freeze-preserved human normal corneal epithelial cells, the cells were seeded according to a conventional method and cultured for 7 days at 37 °C in 5% CO₂. After the cultivation, the cells were harvested by a conventional method, and were suspended in EpiLife (-) at a concentration of 1.0 × 10⁴ cells/mL. The above-described test substance solution was dispensed into a 24 well plate in an amount of 0.5 mL/well. The cell suspension (0.5 mL) was seeded in each well of the 24 well plate (5.0 × 10³ cells/well), and cultivated for 8 days. The culture medium was replaced every 48 hours. As a control, EpiLife (-) to which no test substance was added was used.
   (2) Cell count
      After completion of the cultivation, the supernatant in the 24 well plate was removed, and the plate was washed with 1.0 mL of EpiLife (-) three times. The number of the cells was counted based on the ability to reduce MTT (3-(4,5-dimethyl-2-thiazolyl)-2,5-diphenyl-2H-tetrazolium bromide) according to the protocol of Cell Counting Kit-8 (Dojindo Laboratories). To each well of the 24 well microtiter plate, EpiLife (-) was dispensed in an amount of 400 µL/well, and then 40 µL of the Cell Counting Kit-8 solution was added to each well. The plate was incubated at 37°C in 5% CO₂ for 2 hours and then, the absorbance was measured at 450 nm using a microplate reader.
4. Statistical Analysis
   Average values of absorbance in the control group and each test substance-addition group were calculated, and the values were compared between the control group and each test substance-addition group by a Dunnett's multiple comparison test (one-tailed). The significance level was defined as 5%.
5. Test results
   Fig. 10 shows the results of the cell counting (MTT reduction ability). The vertical axis shows the proportion (%) of the cell number of the test substance-addition group relative to the cell number of the control group. After the cultivation for 8 days, the cell number was increased in the peptide 24-addition group and the PACAP27-addition group, as compared with the control group, and there was a significant difference between the control group and the test substance-addition groups (p < 0.05). As evident from the above-described results, PACAP and a PACAP derivative (peptide 24) affect the corneal epithelial cells and thereby have a promoting effect on the proliferation.

### Test Example 9

### Promoting effect on treatment of rabbit corneal epithelial injury

1. Used animal
Male Japanese white rabbits with a body weight of 2.0 to 2.5 kg purchased from Kitayama Labes Co., Ltd were used. The animals were each put in separate cages placed in a room wherein a temperature of 23 ± 3°C and a humidity of 55 ± 10% were kept and a light was on for 12 hours per day (lighting at 8:00 and lights-out at 20:00), and raised after arrival until the final day of the test. The animals were given 100g of solid feed (Labo R Stock, Nosan Corporation) per day, and could freely have tap water.
2. Test Substance
PACAP27 was used as a test substance. The test substance was dissolved in the following vehicle solution at a concentration of 10 µM or 100µM to prepare eye drops. As a control, the following vehicle solution that does not the test substance was used.

| Formulation of a vehicle solution: | |
|---|---|
| sodium dihydrogen phosphate dihydrate | 0.1g |
| sodium chloride | 0.9g |
| sodium hydroxide | appropriate amount |
| purified water | appropriate amount |
| Total volume | 100 mL (pH 7) |

3. Test method
(1) Creation of corneal flap
   The animal was systemically anesthetized by intramuscular injection (0.9 mL/kg) of a mixture of Ceratal (2% xylazine) and Ketaral (5% ketamine) in a ratio of 0.5:1. After sufficient exposure of the eyeballs, a microkeratome (Nidek Co., Ltd.) was used to create a corneal flap with a diameter of 8.5 mm. For this surgery, an adapter for a rabbit and a blade with a thickness of 130 µm were used.
(2) Creation of corneal epithelial defect
   After creation of the flap, the corneal flap was precisely returned to the original position under a stereoscopic microscope. One week after the surgery, the rabbit was subjected to general anesthesia. Filter paper with a diameter of 6 mm was sufficiently immersed in n-heptanol and then, excess n-heptanol was removed from the filter paper. The filter paper was contacted the center of the cornea for 60 seconds to abrade the corneal epithelium.
(3) Administration by ocular instillation
   The eye drops containing the test substance, or the vehicle solution was continuously administrated by ocular instillation from the day of creation of the corneal flap to the day when the corneal epithelial injury was completely cured. The animals were divided into a vehicle solution-administration (control) group, a 10 µM PACAP27-administartion group and a 100 µM PACAP27-administration group. The eye drops or the vehicle solution was administered 4 times a day at 2-hour intervals in an amount of 50 µL per time. For infection prevention, one drop of 0.3% lomefloxacin eye drops (Lomeflon, Senju Pharmaceutical Co., Ltd.) was administered by ocular instillation simultaneously with administration of the test substance or the vehicle solution for 1 week immediately after the flap creation surgery.
(4) Image analysis
   A defect site of the corneal epithelium was stained by administrating 0.1% fluorescein to the eye immediately after and 18, 24, 42 and 48 hours after abrasion of the corneal epithelium. The stained defect site was photographed using a slit lamp of which flash part was fitted with a cobalt filter. The photograph was stored in a computer as a digital image, and the area of the stained corneal epithelial defect site was measured using image analysis software (Image-Pro Plus, Ver. 4.0, produced by Planetron Inc.).

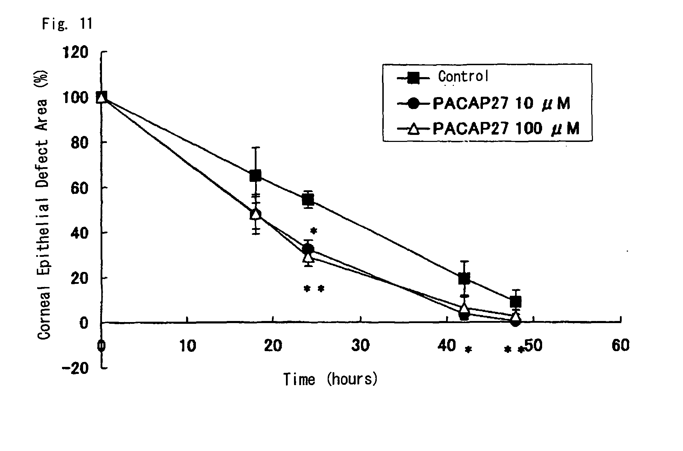

4. Statistical Analysis
The proportion (%) of the corneal epithelial defect area at each measurement time point to the corneal epithelial defect area immediately after creation of the corneal epithelial defect was compared between the control group and the test substance-administration groups using Dunnett's multiple comparison analysis (SAS preclinical package, Version 5.0, SAS Institute Japan). The significance level was defined as less than 5%.
5. Test results
Fig. 11 shows the time course of wound healing of corneal epithelium by administrating PACAP27 to the eye. The area (%) of the remaining corneal epithelial defect at each measurement time point was used index of the wound healing of the corneal epithelium, assuming that the initial area of the corneal epithelial defect was 100%. Although the area of the remaining corneal epithelial defect was gradually reduced with the lapse of time in each group, it was more rapidly reduced in the PACAP27-administration group than in the control group. Significant difference in the proportion of the remaining corneal epithelial defect area between 10 µM PACAP27-administration group and the control group were found at 24 hours, 42 hours and 48 hours after abrasion of the corneal epithelium. A significant difference in the proportion of the remaining corneal epithelial defect area between 100 µM PACAP27-administration group and the control group was found at 24 hours after abrasion of the corneal epithelium. As evident from the above-described results, administration of PACAP27 to the eye brings about a promoting effect on the wound healing of corneal epithelium.

### Example 2

Examples of pharmaceutical preparations are described below.

| Preparation Example 1: Tablet | |
|---|---|
| Peptide 17 | 10 mg |
| Lactose | 80 mg |
| Starch | 17 mg |
| Magnesium stearate | 3 mg |
| Crystalline cellulose | 10 mg |

A tablet was prepared by a conventional method using the above-mentioned ingredients for one tablet. The tablet may be coated with an enteric coating agent (e.g. hydroxypropyl methyl cellulose phthalate or the like), a sugar coating agent or a film (e.g. ethyl cellulose) which is conventionally used, if necessary.

| Preparation Example 2: Capsule | |
|---|---|
| PACAP27 | 50 mg |
| Mannitol | 75 mg |
| Starch | 17 mg |
| Calcium stearate | 3 mg |

The above-mentioned ingredients for one capsule were homogeneously mixed, granulated by a conventional method, and then were filled in a hard capsule. The granules may be coated with an enteric coating agent (e.g. hydroxypropyl methyl cellulose phthalate), a sugar coating agent or a film (e.g. ethyl cellulose) which is conventionally used, if necessary, before they are filled in a capusule.

| Preparation Example 3: Injection | |
|---|---|
| PACAP38 | 750 mg |
| Sodium carboxymethyl cellulose | 500 mg |
| Water for injection | q.s. to 100 mL |

The above-mentioned ingredients were antiseptically mixed to prepare an injection.

| Preparation Example 4: Eye drops | |
|---|---|
| Peptide 17 | 5 mg |
| Boric acid | 700 mg |
| Borax | appropriate amount (pH 7.0) |
| Sodium chloride | 500 mg |
| Hydroxymethyl cellulose | 0.5 g |
| Sodium edetate | 0.05 g |
| Benzalkonium chloride | 0.005 g |
| Sterilized purified water | q.s. to 100 mL |

Sterilized purified water (80 mL) was heated to about 80°C and thereto hydroxymethyl cellulose was added. The mixture was stirred and then cooled to room temperature. To the mixture, peptide 17, sodium chloride, boric acid, sodium edetate and benzalkonium chloride were added and dissolved. The solution was adjusted to pH 7.0 by addition of an appropriate amount of borax. The solution was adjusted to 100 mL with sterilized purified water using a graduated cylinder.

| Preparation Example 5: Eye drops | |
|---|---|
| PACAP27 | 10 mg |
| D-mannitol | 4.5 g |
| Sodium dihydrogen phosphate | 0.1 g |
| Sodium hydroxide | appropriate amount (pH 7.0) |
| Sterilized purified water | q.s. to 100 mL |

PACAP27, D-mannitol and sodium dihydrogen phosphate were dissolved in 80 mL of sterilized purified water. The solution was adjusted to pH 7.0 by addition of an appropriate amount of sodium hydroxide. The solution was adjusted to 100 mL with sterilized purified water using a graduated cylinder. The eye drops thus obtained were filtered with a membrane filter and then filled in a disposable (unit dose) container, and the container was sealed.

| Preparation Example 6: Eye drops | |
|---|---|
| Peptide 24 | 5 mg |
| Boric acid | 700 mg |
| Borax | appropriate amount (pH 7.0) |
| Sodium chloride | 500 mg |
| Hydroxyethyl cellulose | 0.2 g |
| Sodium edetate | 0.05 g |
| Chlorhexidine gluconate | 0.005 g |
| Sterilized purified water | q.s. to 100 mL |

Hydroxymethyl cellulose was added to 80 mL of sterilized purified water which had been heated to about 80°C, stirred and then cooled to room temperature. In the solution, peptide 24, sodium chloride, boric acid, sodium edetate and chlorhexidine gluconate were dissolved. The solution was adjusted to pH 7 by addition of borax. The volume of the solution was adjusted to 100 mL with sterilized purified water using a graduated cylinder.

| Preparation Example 7: Eye drops | |
|---|---|
| Peptide 24 | 0.01 g |
| Sodium dihydrogen phosphate dihydrate | 0.1 g |
| Sodium chloride | 0.9 g |
| Sodium hydroxide | appropriate amount |
| Benzalkonium chloride | 0.005 g |
| Purified water | q.s. to 100 mL |

Sodium dihydrogen phosphate dihydrate, sodium chloride and benzalkonium chloride were sequentially dissolved in about 90 mL of purified water. Peptide 24 was dissolved in the solution. The solution was adjusted to pH 8.0 by adding sodium hydroxide with stirring. The solution was adjusted to 100 mL with purified water using a graduated cylinder, and then sterilized by filtration.

| Preparation Example 8: Eye drops | |
|---|---|
| Peptide 24 | 0.01 g |
| Sodium acetate trihydrate | 0.1 g |
| Sodium chloride | 0.9 g |
| Hydrochloric acid | appropriate amount |
| Benzalkonium chloride | 0.005 g |
| Purified water | q.s. to 100 mL |

Sodium acetate trihydrate, sodium chloride and benzalkonium chloride were sequentially dissolved in about 90 mL of purified water. Peptide 24 was dissolved in the solution. The solution was adjusted to pH 5.0 by adding hydrochloric acid with stirring. The solution was adjusted to 100 mL with purified water using a graduated cylinder, and then sterilized by filtration.

| Preparation Example 9: Eye drops | |
|---|---|
| Peptide 24 | 0.01 g |
| HEPES | 0.1 g |
| Sodium chloride | 0.9 g |
| Sodium hydroxide | appropriate amount |
| Benzalkonium chloride | 0.005 g |
| Purified water | q.s. to 100 mL |

HEPES, sodium chloride and benzalkonium chloride were sequentially dissolved in about 90 mL of purified water. Peptide 24 was dissolved in the solution. The solution was adjusted to pH 7.0 by adding sodium hydroxide with stirring. The solution was adjusted to 100 mL with purified water using a graduated cylinder, and then sterilized by filtration.

### Sequence listing Free Text

SEQ ID No. 1; Xaa is Val or Ile (location: 5)
SEQ ID No. 1 ; Xaa is Asp, Ala or Glu (location: 8)
SEQ ID No. 1; Xaa is Asn or Ser (location: 9)
SEQ ID No. 1; Xaa is Thr or Ser (location: 11)
SEQ ID No. 1; Xaa is Leu or Tyr (location: 13)
SEQ ID No. 1; Xaa is Lys or Arg (location: 15)
SEQ ID No. 1; Xaa is Leu or Nle (location: 17)
SEQ ID No. 1; Xaa is Lys or Arg (location: 20)
SEQ ID No. 1; Xaa is Lys or Arg (location: 21)
SEQ ID No. 1; Xaa is Leu-NH₂ or Leu-OH (location: 23)
SEQ ID No. 2; Xaa is Val or Ile (location: 5)
SEQ ID No. 2; Xaa is Asp, Ala or Glu (location: 8)
SEQ ID No. 2; Xaa is Asn or Ser (location:9)
SEQ ID No. 2; Xaa is The or Ser (location: 11)
SEQ ID No. 2; Xaa is Leu or Tyr (location: 13)
SEQ ID No. 2; Xaa is Lys or Arg (location: 15)
SEQ ID No. 2; Xaa is Leu or Nle (location: 17)
SEQ ID No. 2; Xaa is Lys or Arg (location: 20)
SEQ ID No. 2; Xaa is Lys or Arg (location: 21)
SEQ ID No. 2; Xaa is Ala-NH₂ or Ala-OH (location: 24)
SEQ ID No. 3; Xaa is Val or Ile (location: 5)
SEQ ID No. 3; Xaa is Asp, Ala or Glu (location: 8)
SEQ ID No. 3; Xaa is Asn or Ser (location: 9)
SEQ ID No. 3; Xaa is Thr or Ser (location: 11)
SEQ ID No. 3; Xaa is Leu or Tyr (location: 13)
SEQ ID No. 3; Xaa is Lys or Arg (location: 15)
SEQ ID No. 3: Xaa is Leu or Nle (location: 17)
SEQ ID No. 3; Xaa is Lys or Arg (location: 20)
SEQ ID No. 3; Xaa is Lys or Arg (location: 21)
SEQ ID No. 3: Xaa is Ala--NH₂ or Ala-OH (location: 25)
SEQ ID No. 4; Xaa is Val or Ile (location: 5)
SEQ ID No. 4; Xaa is Asp, Ala or Glu (location: 8)
SEQ ID No. 4; Xaa is Asn or Ser (location: 9)
SEQ ID No. 4; Xaa is Thr or Ser (location: 11)
SEQ ID No. 4; Xaa is Leu or Tyr (location: 13)
SEQ ID No. 4; Xaa is Lys or Arg (location: 15)
SEQ ID No. 4; Xaa is Leu or Nle (location: 17)
SEQ ID No. 4; Xaa is Lys or Arg (location: 20)
SEQ ID No. 4; Xaa is Lys or Arg (location: 21)
SEQ ID No. 4; Xaa is Ile-NH₂, Ile-OH, Val-NH₂ or Val-OH (location: 26)
SEQ ID No. 5; Xaa is Val or Ile (location: 5)
SEQ ID No. 5; Xaa is Asp, Ala or Glu (location: 8)
SEQ ID No. 5; Xaa is Asn or Ser (location: 9)
SEQ ID No. 5; Xaa is Thr or Ser (location: 11)
SEQ ID No. 5; Xaa is Leu or Tyr (location: 13)
SEQ ID No. 5; Xaa is Lys or Arg (location: 15)
SEQ ID No. 5; Xaa is Leu or Nle (location: 17)
SEQ ID No. 5; Xaa is Lys or Arg (location: 20)
SEQ ID No. 5; Xaa is Lys or Arg (location: 21)
SEQ ID No. 5; Xaa is Ile or Val (location: 26)
SEQ ID No. 5; Xaa is Leu-NH₂ or Leu-OH (location: 27)
SEQ ID No. 6; Xaa is Val or Ile (location: 5)
SEQ ID No. 6; Xaa is Asp, Ala or Glu (location: 8)
SEQ ID No. 6; Xaa is Asn or Ser (location: 9)
SEQ ID No. 6; Xaa is Thr or Ser (location: 11)
SEQ ID No. 6; Xaa is Leu or Tyr (location: 13)
SEQ ID No. 6; Xaa is Lys or Arg (location: 15)
SEQ ID No. 6; Xaa is Leu or Nle (location: 17)
SEQ ID No. 6; Xaa is Lys or Arg (location: 20)
SEQ ID No. 6; Xaa is Lys or Arg (location: 21)
SEQ ID No. 6; Xaa is Ile or Val (location: 26)
SEQ ID No. 6; Xaa is Asn-NH₂ or Asn-OH (location: 28)
SEQ ID No. 7; Xaa is Val or Ile (location: 5)
SEQ ID No. 7; Xaa is Asp, Ala or Glu (location: 8)
SEQ ID No. 7; Xaa is Asn or Ser (location: 9)
SEQ ID No. 7; Xaa is Thr or Ser (location: 11)
SEQ ID No. 7; Xaa is Leu or Tyr (location: 13)
SEQ ID No. 7; Xaa is Lys or Arg (location: 15)
SEQ ID No. 7; Xaa is Leu or Nle (location: 17)
SEQ ID No. 7; Xaa is Lys or Arg (location: 20)
SEQ ID No. 7; Xaa is Lys or Arg (location: 21)
SEQ ID No. 7; Xaa is Ile or Val (location: 26)
SEQ ID No. 7; Xaa is Gly-NH₂ or Gly-OH (location: 28)
SEQ ID No. 8; Xaa is Val or Ile (location: 5)
SEQ ID No. 8; Xaa is Asp, Ala or Glu (location: 8)
SEQ ID No. 8; Xaa is Asn or Ser (location: 9)
SEQ ID No. 8; Xaa is Thr or Ser (location: 11)
SEQ ID No. 8; Xaa is Leu or Tyr (location: 13)
SEQ ID No. 8; Xaa is Lys or Arg (location: 15)
SEQ ID No. 8; Xaa is Leu or Nle (location: 17)
SEQ ID No. 8; Xaa is Lys or Arg (location: 20)
SEQ ID No. 8; Xaa is Lys or Arg (location: 21)
SEQ ID No. 8; Xaa is Ile or Val (location: 26)
SEQ ID No. 8; Xaa is Lys-NH₂ or Lys-OH (location: 29)
SEQ ID No. 9; Xaa is Val or Ile (location: 5)
SEQ ID No. 9; Xaa is Asp, Ala or Glu (location: 8)
SEQ ID No. 9; Xaa is Asn or Ser (location: 9)
SEQ ID No. 9; Xaa is Thr or Ser (location: 11)
SEQ ID No. 9; Xaa is Leu or Tyr (location: 13)
SEQ ID No. 9; Xaa is Lys or Arg (location: 15)
SEQ ID No. 9; Xaa is Leu or Nle (location: 17)
SEQ ID No. 9; Xaa is Lys or Arg (location: 20)
SEQ ID No. 9; Xaa is Lys or Arg (location: 21)
SEQ ID No. 9; Xaa is Ile or Val (location: 26)
SEQ ID No. 9; Xaa is Arg-NH₂ or Arg-OH (location: 27)
SEQ ID No. 10; Xaa is Val or Ile (location: 5)
SEQ ID No. 10; Xaa is Asp, Ala or Glu (location: 8)
SEQ ID No. 10; Xaa is Asn or Ser (location: 9)
SEQ ID No. 10; Xaa is Thr or Ser (location: 11)
SEQ ID No. 10; Xaa is Leu or Tyr (location: 13)
SEQ ID No. 10; Xaa is Lys or Arg (location: 15)
SEQ ID No. 10; Xaa is Leu or Nle (location: 17)
SEQ ID No. 10; Xaa is Lys or Arg (location: 20)
SEQ ID No. 10; Xaa is Lys or Arg (location: 21)
SEQ ID No. 10; Xaa is Ile or Val (location: 26)
SEQ ID No. 10; Xaa is Lys-NH₂ or Lys-OH (location: 30)
SEQ ID No. 11; Xaa is Val or Ile (location: 5)
SEQ ID No. 11; Xaa is Asp, Ala or Glu (location: 8)
SEQ ID No. 11; Xaa is Asn or Ser (location: 9)
SEQ ID No. 11; Xaa is Thr or Ser (location: 11)
SEQ ID No. 11; Xaa is Leu or Tyr (location: 13)
SEQ ID No. 11; Xaa is Lys or Arg (location: 15)
SEQ ID No. 11; Xaa is Leu or Nle (location: 17)
SEQ ID No. 11; Xaa is Lys or Arg (location: 20)
SEQ ID No. 11; Xaa is Lys or Arg (location: 21)
SEQ ID No. 11; Xaa is Ile or Val (location: 26)
SEQ ID No. 11; Xaa is Arg-NH₂ or Arg-OH (location: 30)
SEQ ID No. 12; Xaa is Val or Ile (location: 5)
SEQ ID No. 12; Xaa is Asp, Ala or Glu (location: 8)
SEQ ID No. 12; Xaa is Asn or Ser (location: 9)
SEQ ID No. 12; Xaa is Thr or Ser (location: 11)
SEQ ID No. 12; Xaa is Leu or Tyr (location: 13)
SEQ ID No. 12; Xaa is Lys or Arg (location: 15)
SEQ ID No. 12; Xaa is Leu or Nle (location: 17)
SEQ ID No. 12; Xaa is Lys or Arg (location: 20)
SEQ ID No. 12; Xaa is Lys or Arg (location: 21)
SEQ ID No. 12; Xaa is Ile or Val (location: 26)
SEQ ID No. 12; Xaa is Arg-NH₂ or Arg-OH (location: 30)
SEQ ID No. 13; Xaa is Val or Ile (location s: 5)
SEQ ID No. 13; Xaa is Asp, Ala or Glu (location: 8)
SEQ ID No. 13; Xaa is Asn or Ser (location: 9)
SEQ ID No. 13; Xaa is Thr or Ser (location: 11)
SEQ ID No. 13; Xaa is Leu or Tyr (location: 13)
SEQ ID No. 13; Xaa is Lys or Arg (location: 15)
SEQ ID No. 13; Xaa is Leu or Nle (location: 17)
SEQ ID No. 13; Xaa is Lys or Arg (location: 20)
SEQ ID No. 13; Xaa is Lys or Arg (location: 21)
SEQ ID No. 13; Xaa is Ile or Val (location: 26)
SEQ ID No. 13; Xaa is Lys-NH₂ or Lys-OH (location: 38)
SEQ ID No. 14; Xaa is Val or Ile (location: 5)
SEQ ID No. 14; Xaa is Asp, Ala or Glu (location: 8)
SEQ ID No. 14; Xaa is Asn or Ser (location: 9)
SEQ ID No. 14; Xaa is Thr or Ser (location: 11)
SEQ ID No. 14; Xaa is Leu or Tyr (location: 13)
SEQ ID No. 14; Xaa is Lys or Arg (location: 15)
SEQ ID No. 14; Xaa is Leu or Nle (location: 17)
SEQ ID No. 14; Xaa is Lys or Arg (location: 20)
SEQ ID No. 14; Xaa is Lys or Arg (location: 21)
SEQ ID No. 14; Xaa is Ile or Val (location: 26)
SEQ ID No. 14; Xaa is Arg-NH₂ or Arg-OH (location: 38)
SEQ ID No. 15; Xaa is Leu-NH₂ (location: 27)
SEQ ID No. 16; Xaa is Lys-NH₂ (location: 38)
SEQ ID No. 17; Xaa is Leu-NH₂ (location: 23)
SEQ ID No. 18; Xaa is Asn-NH₂ (location: 28)
SEQ ID No. 19; Xaa is Arg-NH₂ (location: 30)
SEQ ID No. 20; Xaa is Acetyl-His (location: 1)
SEQ ID No. 20; Xaa is Arg-NH₂ (location: 30)
SEQ ID No. 21; Xaa is Stearoyl-His (location: 1)
SEQ ID No. 21; Xaa is Arg-NH₂ (location: 30)
SEQ ID No. 22; Xaa is Arg-OH (location: 30)
SEQ ID No. 23; Xaa is Nle (location: 17)
SEQ ID No. 23; Xaa is Arg-NH₂ (location: 30)
SEQ ID No. 24; Xaa is Gly-NH₂ (location: 28)
SEQ ID No. 25; Xaa is Lys-NH₂ (location: 29)
SEQ ID No. 26; Xaa is Arg-NH₂ (location: 29)
SEQ ID No. 27; Xaa is Arg-NH₂ (location: 30)
SEQ ID No. 28; Xaa is Arg-NH₂ (location: 30)
SEQ ID No. 29; Xaa is Leu-NH₂ (location: 23)
SEQ ID No. 30; Xaa is Arg-NH₂ (location: 30)
SEQ ID No. 31; Xaa is Leu-NH₂ (location: 23)
SEQ ID No. 32; Xaa is Arg-NH₂ (location: 30)
SEQ ID No. 33; Xaa is Arg-OH (location: 30)
SEQ ID No. 34; Xaa is Arg-NH₂ (location: 30)
SEQ ID No. 35; Xaa is Acetyl-His (location: 1)
SEQ ID No. 35; Xaa is Arg-NH₂ (location: 30)
SEQ ID No. 36; Xaa is Arg-NH₂ (location: 38)
SEQ ID No. 37; Xaa is Arg-NH₂ (location: 38)
SEQ ID No. 38; Xaa is Leu-NH₂ (location: 23)
SEQ ID No. 39; Xaa is Acetyl-His (location: 1)
SEQ ID No. 39; Xaa is Leu-NH₂ (location: 23)
SEQ ID No. 40; Xaa is Arg-NH₂ (location: 30)

## Claims

1. An agent for promoting corneal neuritogenesis comprising PACAP, a PACAP derivative or a pharmaceutically acceptable salt thereof.

2. An agent for improving corneal sensitivity comprising PACAP, a PACAP derivative or a pharmaceutically acceptable salt thereof.

3. A therapeutic agent for dry eyes comprising PACAP, a PACAP derivative or a pharmaceutically acceptable salt thereof.

4. A therapeutic agent for corneal epithelial injury comprising PACAP, a PACAP derivative or a pharmaceutically acceptable salt thereof.

5. The agent for promoting corneal neuritogenesis according to Claim 1, wherein the PACAP derivative is a peptide (SEQ ID Nos. 1 to 14) comprising at least 23 residues from the N-terminal of a peptide represented by the following formula (I):
His-Ser-Asp-Ala-X1-Phe-Thr-X2-X3-Tyr-X4-Arg-X5-Arg-X6-Gln-X7-Ala-Val-X8-X9-Tyr-Leu-Ala-Ala-X10-X11-X12 (I)
wherein,
X1 represents Val or Ile;
X2 represents Asp, Ala or Glu;
X3 represents Asn or Ser;
X4 represents Thr or Ser;
X5 represents Leu or Tyr;
X6, X8 and X9 represent Lys or Arg;
X7 represents Leu or Nle;
X10 represents Ile, Val or a chemical bond;
X11 represents Leu, Leu-Asn, Leu-Gly, Leu-Gly-Lys, Leu-Gly-Arg, Leu-Gly-Lys-Lys, Leu-Gly-Lys-Arg, Leu-Gly-Arg-Arg, Leu-Gly-Lys-Arg-Tyr-Lys-Gln-Arg-Val-Lys-Asn-Lys, Leu-Gly-Arg-Arg-Tyr-Arg-Gln-Arg-Val-Arg-Asn-Arg, or a chemical bond; and
X12 modifies the α-carboxyl group of the C-terminal amino acid, and represents -NH₂ or -OH;
or pharmaceutically acceptable salts thereof.

6. The agent for improving corneal sensitivity according to Claim 2, wherein the PACAP derivative is a peptide comprising at least 23 residues from the N-terminal of a peptide represented by the above formula (I) or a pharmaceutically acceptable salt thereof.

7. The therapeutic agent for dry eyes according to Claim 3, wherein the PACAP derivative is a peptide comprising at least 23 residues from the N-terminal of a peptide represented by the above formula (I) or a pharmaceutically acceptable salt thereof.

8. The therapeutic agent for corneal epithelial injury according to Claim 4, wherein the PACAP derivative is a peptide comprising at least 23 residues from the N-terminal of a peptide represented by the above formula (I) or a pharmaceutically acceptable salt thereof.

9. The agent for promoting corneal neuritogenesis according to Claim 5, wherein the PACAP derivative is a peptide represented by the above formula (I) in which X1 is Ile, X2 is Asp, X3 is Ser, X4 is Ser, X5 is Tyr, X6, X8 and X9 are Arg, X7 is Leu, X10 is Val and X11 is Leu-Gly-Arg-Arg.

10. The agent for improving corneal sensitivity according to Claim 6, wherein the PACAP derivative is a peptide represented by the above formula (I) in which X1 is Ile, X2 is Asp, X3 is Ser, X4 is Ser, X5 is Tyr, X6, X8 and X9 are Arg, X7 is Leu, X10 is Val and X11 is Leu-Gly-Arg-Arg.

11. The therapeutic agent for dry eyes according to Claim 7, wherein the PACAP derivative is a peptide represented by the above formula (I) in which X1 is Ile, X2 is Asp, X3 is Ser, X4 is Ser, X5 is Tyr, X6, X8 and X9 are Arg, X7 is Leu, X10 is Val and X11 is Leu-Gly-Arg-Arg.

12. The therapeutic agent for corneal injury according to Claim 8, wherein the PACAP derivative is a peptide represented by the above formula (I) in which X1 is Ile, X2 is Asp, X3 is Ser, X4 is Ser, X5 is Tyr, X6, X8 and X9 are Arg, X7 is Leu, X10 is Val and X11 is Leu-Gly-Arg-Arg.

13. Use of PACAP, a PACAP derivative or a pharmaceutically acceptable salt thereof in the production of an agent for promoting corneal neuritogenesis.

14. The use according to Claim 13, wherein the PACAP derivative is a peptide comprising at least 23 residues from the N-terminal of a peptide represented by the above formula (I) or a pharmaceutically acceptable salt thereof.

15. Use of PACAP, a PACAP derivative or a pharmaceutically acceptable salt thereof in the production of an agent for improving corneal sensitivity.

16. The use according to Claim 15, wherein the PACAP derivative is a peptide comprising at least 23 residues from the N-terminal of a peptide represented by the above formula (I) or a pharmaceutically acceptable salt thereof.

17. Use of PACAP, a PACAP derivative or a pharmaceutically acceptable salt thereof in the production of a therapeutic agent for dry eyes.

18. The use according to Claim 17, wherein the PACAP derivative is a peptide comprising at least 23 residues from the N-terminal of a peptide represented by the above formula (I) or a pharmaceutically acceptable salt thereof.

19. Use of PACAP, a PACAP derivative or a pharmaceutically acceptable salt thereof in the production of a therapeutic agent for corneal epithelial injury.

20. The use according to Claim 19, wherein the PACAP derivative is a peptide comprising at least 23 residues from the N-terminal of a peptide represented by the above formula (I) or a pharmaceutically acceptable salt thereof.

21. A method for promoting corneal neuritogenesis which comprises administering an effective amount of PACAP, a PACAP derivative or a pharmaceutically acceptable salt thereof to a subject in need thereof.

22. The method according to Claim 21, wherein the PACAP derivative is a peptide comprising at least 23 residues from the N-terminal of a peptide represented by the above formula (I) or a pharmaceutically acceptable salt thereof.

23. A method for improving corneal sensitivity which comprises administering an effective amount of PACAP, a PACAP derivative or a pharmaceutically acceptable salt thereof to a subject in need thereof.

24. The method according to Claim 23, wherein the PACAP derivative is a peptide comprising at least 23 residues from the N-terminal of a peptide represented by the above formula (I) or a pharmaceutically acceptable salt thereof.

25. A method for treating dry eyes which comprises administering an effective amount of PACAP, a PACAP derivative or a pharmaceutically acceptable salt thereof to a subject in need thereof.

26. The method according to Claim 25, wherein the PACAP derivative is a peptide comprising at least 23 residues from the N-terminal of a peptide represented by the above formula (I) or a pharmaceutically acceptable salt thereof.

27. A method for treating corneal epithelial injury which comprises administering an effective amount of PACAP, a PACAP derivative or a pharmaceutically acceptable salt thereof to a subject in need thereof.

28. The method according to Claim 27, wherein the PACAP derivative is a peptide comprising at least 23 residues from the N-terminal of a peptide represented by the above formula (I) or a pharmaceutically acceptable salt thereof.


SEQUENCE LISTING

<110> SENJU PHARMACEUTICAL CO., LTD. and
ITOHAM FOODS INC.

<120> AGENT FOR PROMOTING FORMATION CORNEAL NEURITE COMPRISING PACAP AND
PACAP DERIVATIVES

<130> 665199

<150> JP 2004-128581
<150> JP 2004-330464

<151> 2004-04-23
<151> 2004-11-15

<160> 40

<170> PatentIn version 3.1

<210> 1
<211> 23
<212> PRT
<213> Artificial Sequence

<220>
<223> Synthetic Peptide

<220>
<221> MISC_FEATURE
<222> (5)..(5)
<223> Xaa=Val or Ile

<220>
<221> MISC_FEATURE
<222> (8)..(8)
<223> Xaa=Asp, Ala or Glu

<220>
<221> MISC_FEATURE
<222> (9)..(9)

<223>  Xaa=Asn or Ser

<220>
<221>  MISC_FEATURE
<222>  (11)..(11)
<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)
<223>  Xaa=Lys or Arg

<220>
<221>  MISC_FEATURE
<222>  (17)..(17)
<223>  Xaa=Leu or Nle

<220>
<221>  MISC_FEATURE
<222>  (20)..(20)
<223>  Xaa=Lys or Arg

<220>
<221>  MISC_FEATURE
<222>  (21)..(21)
<223>  Xaa=Lys or Arg

<220>
<221>  MOD_RES
<222>  (23)..(23)
<223>  Xaa=Leu-NH2 or Leu-OH

<400>  1

His Ser Asp Ala Xaa Phe Thr Xaa Xaa Tyr Xaa Arg Xaa Arg Xaa Gln

1               5                   10                  15

Xaa Ala Val Xaa Xaa Tyr Xaa
            20

<210>  2
<211>  24
<212>  PRT
<213>  Artificial Sequence

<220>
<223>  Synthetic Peptide

<220>
<221>  MISC_FEATURE
<222>  (5)..(5)
<223>  Xaa=Val or Ile

<220>
<221>  MISC_FEATURE
<222>  (8)..(8)
<223>  Xaa=Asp, Ala or Glu

<220>
<221>  MISC_FEATURE
<222>  (9)..(9)
<223>  Xaa=Asn or Ser

<220>
<221>  MISC_FEATURE
<222>  (11)..(11)
<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)

<223>  Xaa=Lys or Arg

<220>
<221>  MISC_FEATURE
<222>  (17)..(17)
<223>  Xaa=Leu or Nle

<220>
<221>  MISC_FEATURE
<222>  (20)..(20)
<223>  Xaa=Lys or Arg

<220>
<221>  MISC_FEATURE
<222>  (21)..(21)
<223>  Xaa=Lys or Arg

<220>
<221>  MOD_RES
<222>  (24)..(24)
<223>  Xaa=Ala-NH2 or Ala-OH

<400>  2

His Ser Asp Ala Xaa Phe Thr Xaa Xaa Tyr Xaa Arg Xaa Arg Xaa Gln
1               5                   10                  15

Xaa Ala Val Xaa Xaa Tyr Leu Xaa
            20

<210>  3
<211>  25
<212>  PRT
<213>  Artificial Sequence

<220>
<223>  Synthetic Peptide

<220>
<221>  MISC_FEATURE
<222>  (5)..(5)

<223>  Xaa=Val or Ile

<220>
<221>  MISC_FEATURE
<222>  (8)..(8)
<223>  Xaa=Asp, Ala or Glu

<220>
<221>  MISC_FEATURE
<222>  (9)..(9)
<223>  Xaa=Asn or Ser

<220>
<221>  MISC_FEATURE
<222>  (11)..(11)
<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)
<223>  Xaa=Lys or Arg

<220>
<221>  MISC_FEATURE
<222>  (17)..(17)
<223>  Xaa=Leu or Nle

<220>
<221>  MISC_FEATURE
<222>  (20)..(20)
<223>  Xaa=Lys or Arg

<220>
<221>  MISC_FEATURE
<222>  (21)..(21)

<223>  Xaa=Lys or Arg

<220>
<221>  MOD_RES
<222>  (25)..(25)
<223>  Xaa=Ala-NH2 or Ala-OH

<400>  3

His Ser Asp Ala Xaa Phe Thr Xaa Xaa Tyr Xaa Arg Xaa Arg Xaa Gln
1               5                   10                  15

Xaa Ala Val Xaa Xaa Tyr Leu Ala Xaa
            20                  25

<210>  4
<211>  26
<212>  PRT
<213>  Artificial Sequence

<220>
<223>  Synthetic Peptide

<220>
<221>  MISC_FEATURE
<222>  (5)..(5)
<223>  Xaa=Val or Ile

<220>
<221>  MISC_FEATURE
<222>  (8)..(8)
<223>  Xaa=Asp, Ala or Glu

<220>
<221>  MISC_FEATURE
<222>  (9)..(9)
<223>  Xaa=Asn or Ser

<220>
<221>  MISC_FEATURE
<222>  (11)..(11)

<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)
<223>  Xaa=Lys or Arg

<220>
<221>  MISC_FEATURE
<222>  (17)..(17)
<223>  Xaa=Leu or Nle

<220>
<221>  MISC_FEATURE
<222>  (20)..(20)
<223>  Xaa=Lys or Arg

<220>
<221>  MISC_FEATURE
<222>  (21)..(21)
<223>  Xaa=Lys or Arg

<220>
<221>  MOD_RES
<222>  (26)..(26)
<223>  Xaa=Ile-NH2, Ile-OH, Val-NH2 or Val-OH

<400>  4

His Ser Asp Ala Xaa Phe Thr Xaa Xaa Tyr Xaa Arg Xaa Arg Xaa Gln
1               5                   10                  15

Xaa Ala Val Xaa Xaa Tyr Leu Ala Ala Xaa
            20                  25

<210> 5
<211> 27
<212> PRT
<213> Artificial Sequence

<220>
<223> Synthetic Peptide

<220>
<221> MISC_FEATURE
<222> (5)..(5)
<223> Xaa=Val or Ile

<220>
<221> MISC_FEATURE
<222> (8)..(8)
<223> Xaa=Asp, Ala or Glu

<220>
<221> MISC_FEATURE
<222> (9)..(9)
<223> Xaa=Asn or Ser

<220>
<221> MISC_FEATURE
<222> (11)..(11)
<223> Xaa=Thr or Ser

<220>
<221> MISC_FEATURE
<222> (13)..(13)
<223> Xaa=Leu or Tyr

<220>
<221> MISC_FEATURE
<222> (15)..(15)
<223> Xaa=Lys or Arg

<220>
<221> MISC_FEATURE
<222> (17)..(17)

<223>  Xaa=Leu or Nle


<220>
<221>  MISC_FEATURE
<222>  (20)..(20)
<223>  Xaa=Lys or Arg


<220>
<221>  MISC_FEATURE
<222>  (21)..(21)
<223>  Xaa=Lys or Arg


<220>
<221>  MOD_RES
<222>  (26)..(26)
<223>  Xaa=Ile or Val


<220>
<221>  MOD_RES
<222>  (27)..(27)
<223>  Xaa=Leu-NH2 or Leu-OH



<400>  5

His Ser Asp Ala Xaa Phe Thr Xaa Xaa Tyr Xaa Arg Xaa Arg Xaa Gln
1               5                   10                  15


Xaa Ala Val Xaa Xaa Tyr Leu Ala Ala Xaa Xaa
            20                  25


<210>  6
<211>  28
<212>  PRT
<213>  Artificial Sequence

<220>
<223>  Synthetic Peptide

<220>
<221>  MISC_FEATURE

<222>  (5)..(5)
<223>  Xaa=Val or Ile


<220>
<221>  MISC_FEATURE
<222>  (8)..(8)
<223>  Xaa=Asp, Ala or Glu


<220>
<221>  MISC_FEATURE
<222>  (9)..(9)
<223>  Xaa=Asn or Ser


<220>
<221>  MISC_FEATURE
<222>  (11)..(11)
<223>  Xaa=Thr or Ser


<220>
<221>  MISC_FEATURE
<222>  (13)..(13)
<223>  Xaa=Leu or Tyr


<220>
<221>  MISC_FEATURE
<222>  (15)..(15)
<223>  Xaa=Lys or Arg


<220>
<221>  MISC_FEATURE
<222>  (17)..(17)
<223>  Xaa=Leu or Nle


<220>
<221>  MISC_FEATURE
<222>  (20)..(20)
<223>  Xaa=Lys or Arg


<220>

<221>  MISC_FEATURE
<222>  (21)..(21)
<223>  Xaa=Lys or Arg


<220>
<221>  MOD_RES
<222>  (26)..(26)
<223>  Xaa=Ile or Val


<220>
<221>  MOD_RES
<222>  (28)..(28)
<223>  Xaa=Asn-NH2 or Asn-OH


<400>  6

His Ser Asp Ala Xaa Phe Thr Xaa Xaa Tyr Xaa Arg Xaa Arg Xaa Gln
1               5                   10                  15


Xaa Ala Val Xaa Xaa Tyr Leu Ala Ala Xaa Leu Xaa
            20                  25


<210>  7
<211>  28
<212>  PRT
<213>  Artificial Sequence

<220>
<223>  Synthetic Peptide

<220>
<221>  MISC_FEATURE
<222>  (5)..(5)
<223>  Xaa=Val or Ile


<220>
<221>  MISC_FEATURE
<222>  (8)..(8)
<223>  Xaa=Asp, Ala or Glu


<220>

<221>  MISC_FEATURE
<222>  (9)..(9)
<223>  Xaa=Asn or Ser

<220>
<221>  MISC_FEATURE
<222>  (11)..(11)
<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)
<223>  Xaa=Lys or Arg

<220>
<221>  MISC_FEATURE
<222>  (17)..(17)
<223>  Xaa=Leu or Nle

<220>
<221>  MISC_FEATURE
<222>  (20)..(20)
<223>  Xaa=Lys or Arg

<220>
<221>  MISC_FEATURE
<222>  (21)..(21)
<223>  Xaa=Lys or Arg

<220>
<221>  MOD_RES
<222>  (26)..(26)
<223>  Xaa=Ile or Val

<220>

<221>  MOD_RES
<222>  (28)..(28)
<223>  Xaa=Gly-NH2 or Gly-OH

<400>  7

His Ser Asp Ala Xaa Phe Thr Xaa Xaa Tyr Xaa Arg Xaa Arg Xaa Gln
1               5                   10                  15

Xaa Ala Val Xaa Xaa Tyr Leu Ala Ala Xaa Leu Xaa
            20                  25

<210>  8
<211>  29
<212>  PRT
<213>  Artificial Sequence

<220>
<223>  Synthetic Peptide

<220>
<221>  MISC_FEATURE
<222>  (5)..(5)
<223>  Xaa=Val or Ile

<220>
<221>  MISC_FEATURE
<222>  (8)..(8)
<223>  Xaa=Asp, Ala or Glu

<220>
<221>  MISC_FEATURE
<222>  (9)..(9)
<223>  Xaa=Asn or Ser

<220>
<221>  MISC_FEATURE
<222>  (11)..(11)
<223>  Xaa=Thr or Ser

<220>

<221>  MISC_FEATURE
<222>  (13)..(13)
<223>  Xaa=Leu or Tyr


<220>
<221>  MISC_FEATURE
<222>  (15)..(15)
<223>  Xaa=Lys or Arg


<220>
<221>  MISC_FEATURE
<222>  (17)..(17)
<223>  Xaa=Leu or Nle


<220>
<221>  MISC_FEATURE
<222>  (20)..(20)
<223>  Xaa=Lys or Arg


<220>
<221>  MISC_FEATURE
<222>  (21)..(21)
<223>  Xaa=Lys or Arg


<220>
<221>  MOD_RES
<222>  (26)..(26)
<223>  Xaa=Ile or Val


<220>
<221>  MOD_RES
<222>  (29)..(29)
<223>  Xaa=Lys-NH2 or Lys-OH


<400>  8

His Ser Asp Ala Xaa Phe Thr Xaa Xaa Tyr Xaa Arg Xaa Arg Xaa Gln
1               5                   10                  15


Xaa Ala Val Xaa Xaa Tyr Leu Ala Ala Xaa Leu Gly Xaa

20                    25

<210>  9
<211>  29
<212>  PRT
<213>  Artificial Sequence

<220>
<223>  Synthetic Peptide

<220>
<221>  MISC_FEATURE
<222>  (5)..(5)
<223>  Xaa=Val or Ile

<220>
<221>  MISC_FEATURE
<222>  (8)..(8)
<223>  Xaa=Asp, Ala or Glu

<220>
<221>  MISC_FEATURE
<222>  (9)..(9)
<223>  Xaa=Asn or Ser

<220>
<221>  MISC_FEATURE
<222>  (11)..(11)
<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)
<223>  Xaa=Lys or Arg

<220>

<221>  MISC_FEATURE
<222>  (17)..(17)
<223>  Xaa=Leu or Nle


<220>
<221>  MISC_FEATURE
<222>  (20)..(20)
<223>  Xaa=Lys or Arg


<220>
<221>  MISC_FEATURE
<222>  (21)..(21)
<223>  Xaa=Lys or Arg


<220>
<221>  MOD_RES
<222>  (26)..(26)
<223>  Xaa=Ile or Val


<220>
<221>  MOD_RES
<222>  (29)..(29)
<223>  Xaa=Arg-NH2 or Arg-OH


<400>  9

His Ser Asp Ala Xaa Phe Thr Xaa Xaa Tyr Xaa Arg Xaa Arg Xaa Gln
1               5                   10                  15


Xaa Ala Val Xaa Xaa Tyr Leu Ala Ala Xaa Leu Gly Xaa
            20                  25


<210>  10
<211>  30
<212>  PRT
<213>  Artificial Sequence

<220>
<223>  Synthetic Peptide

<220>

<221>  MISC_FEATURE
<222>  (5)..(5)
<223>  Xaa=Val or Ile

<220>
<221>  MISC_FEATURE
<222>  (8)..(8)
<223>  Xaa=Asp, Ala or Glu

<220>
<221>  MISC_FEATURE
<222>  (9)..(9)
<223>  Xaa=Asn or Ser

<220>
<221>  MISC_FEATURE
<222>  (11)..(11)
<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)
<223>  Xaa=Lys or Arg

<220>
<221>  MISC_FEATURE
<222>  (17)..(17)
<223>  Xaa=Leu or Nle

<220>
<221>  MISC_FEATURE
<222>  (20)..(20)
<223>  Xaa=Lys or Arg

<220>

<221>  MISC_FEATURE
<222>  (21)..(21)
<223>  Xaa=Lys or Arg


<220>
<221>  MOD_RES
<222>  (26)..(26)
<223>  Xaa=Ile or Val


<220>
<221>  MOD_RES
<222>  (30)..(30)
<223>  Xaa=Lys-NH2 or Lys-OH


<400>  10

His Ser Asp Ala Xaa Phe Thr Xaa Xaa Tyr Xaa Arg Xaa Arg Xaa Gln
1               5                   10                  15


Xaa Ala Val Xaa Xaa Tyr Leu Ala Ala Xaa Leu Gly Lys Xaa
            20                  25


<210>  11
<211>  30
<212>  PRT
<213>  Artificial Sequence

<220>
<223>  Synthetic Peptide

<220>
<221>  MISC_FEATURE
<222>  (5)..(5)
<223>  Xaa=Val or Ile


<220>
<221>  MISC_FEATURE
<222>  (8)..(8)
<223>  Xaa=Asp, Ala or Glu


<220>

<221>  MISC_FEATURE
<222>  (9)..(9)
<223>  Xaa=Asn or Ser


<220>
<221>  MISC_FEATURE
<222>  (11)..(11)
<223>  Xaa=Thr or Ser


<220>
<221>  MISC_FEATURE
<222>  (13)..(13)
<223>  Xaa=Leu or Tyr


<220>
<221>  MISC_FEATURE
<222>  (15)..(15)
<223>  Xaa=Lys or Arg


<220>
<221>  MISC_FEATURE
<222>  (17)..(17)
<223>  Xaa=Leu or Nle


<220>
<221>  MISC_FEATURE
<222>  (20)..(20)
<223>  Xaa=Lys or Arg


<220>
<221>  MISC_FEATURE
<222>  (21)..(21)
<223>  Xaa=Lys or Arg


<220>
<221>  MOD_RES
<222>  (26)..(26)
<223>  Xaa=Ile or Val


<220>

<221>  MOD_RES
<222>  (30)..(30)
<223>  Xaa=Arg-NH2 or Arg-OH


<400>  11

His Ser Asp Ala Xaa Phe Thr Xaa Xaa Tyr Xaa Arg Xaa Arg Xaa Gln
1               5                   10                  15


Xaa Ala Val Xaa Xaa Tyr Leu Ala Ala Xaa Leu Gly Lys Xaa
            20                  25


<210>  12
<211>  30
<212>  PRT
<213>  Artificial Sequence

<220>
<223>  Synthetic Peptide


<220>
<221>  MISC_FEATURE
<222>  (5)..(5)
<223>  Xaa=Val or Ile


<220>
<221>  MISC_FEATURE
<222>  (8)..(8)
<223>  Xaa=Asp, Ala or Glu


<220>
<221>  MISC_FEATURE
<222>  (9)..(9)
<223>  Xaa=Asn or Ser


<220>
<221>  MISC_FEATURE
<222>  (11)..(11)
<223>  Xaa=Thr or Ser

<220>
<221> MISC_FEATURE
<222> (13)..(13)
<223> Xaa=Leu or Tyr

<220>
<221> MISC_FEATURE
<222> (15)..(15)
<223> Xaa=Lys or Arg

<220>
<221> MISC_FEATURE
<222> (17)..(17)
<223> Xaa=Leu or Nle

<220>
<221> MISC_FEATURE
<222> (20)..(20)
<223> Xaa=Lys or Arg

<220>
<221> MISC_FEATURE
<222> (21)..(21)
<223> Xaa=Lys or Arg

<220>
<221> MOD_RES
<222> (26)..(26)
<223> Xaa=Ile or Val

<220>
<221> MOD_RES
<222> (30)..(30)
<223> Xaa=Arg-NH2 or Arg-OH

<400> 12

His Ser Asp Ala Xaa Phe Thr Xaa Xaa Tyr Xaa Arg Xaa Arg Xaa Gln
1               5                   10                  15

Xaa Ala Val Xaa Xaa Tyr Leu Ala Ala Xaa Leu Gly Arg Xaa
            20                      25

<210>  13
<211>  38
<212>  PRT
<213>  Artificial Sequence

<220>
<223>  Synthetic Peptide

<220>
<221>  MISC_FEATURE
<222>  (5)..(5)
<223>  Xaa=Val or Ile

<220>
<221>  MISC_FEATURE
<222>  (8)..(8)
<223>  Xaa=Asp, Ala or Glu

<220>
<221>  MISC_FEATURE
<222>  (9)..(9)
<223>  Xaa=Asn or Ser

<220>
<221>  MISC_FEATURE
<222>  (11)..(11)
<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)
<223>  Xaa=Lys or Arg

<220>
<221> MISC_FEATURE
<222> (17)..(17)
<223> Xaa=Leu or Nle

<220>
<221> MISC_FEATURE
<222> (20)..(20)
<223> Xaa=Lys or Arg

<220>
<221> MISC_FEATURE
<222> (21)..(21)
<223> Xaa=Lys or Arg

<220>
<221> MOD_RES
<222> (26)..(26)
<223> Xaa=Ile or Val

<220>
<221> MOD_RES
<222> (38)..(38)
<223> Xaa=Lys-NH2 or Lys-OH

<400> 13

His Ser Asp Ala Xaa Phe Thr Xaa Xaa Tyr Xaa Arg Xaa Arg Xaa Gln
1               5                   10                  15

Xaa Ala Val Xaa Xaa Tyr Leu Ala Ala Xaa Leu Gly Lys Arg Tyr Lys
            20                  25                  30

Gln Arg Val Lys Asn Xaa
        35

<210> 14
<211> 38
<212> PRT
<213> Artificial Sequence

<220>
<223>  Synthetic Peptide

<220>
<221>  MISC_FEATURE
<222>  (5)..(5)
<223>  Xaa=Val or Ile

<220>
<221>  MISC_FEATURE
<222>  (8)..(8)
<223>  Xaa=Asp, Ala or Glu

<220>
<221>  MISC_FEATURE
<222>  (9)..(9)
<223>  Xaa=Asn or Ser

<220>
<221>  MISC_FEATURE
<222>  (11)..(11)
<223>  Xaa=Thr or Ser

<220>
<221>  MISC_FEATURE
<222>  (13)..(13)
<223>  Xaa=Leu or Tyr

<220>
<221>  MISC_FEATURE
<222>  (15)..(15)
<223>  Xaa=Lys or Arg

<220>
<221>  MISC_FEATURE
<222>  (17)..(17)
<223>  Xaa=Leu or Nle

<220>
<221>  MISC_FEATURE
<222>  (20)..(20)

<223>  Xaa=Lys or Arg

<220>
<221>  MISC_FEATURE
<222>  (21)..(21)
<223>  Xaa=Lys or Arg

<220>
<221>  MOD_RES
<222>  (26)..(26)
<223>  Xaa=Ile or Val

<220>
<221>  MOD_RES
<222>  (38)..(38)
<223>  Xaa=Arg-NH2 or Arg-OH

<400>  14

His Ser Asp Ala Xaa Phe Thr Xaa Xaa Tyr Xaa Arg Xaa Arg Xaa Gln
1               5                   10                  15

Xaa Ala Val Xaa Xaa Tyr Leu Ala Ala Xaa Leu Gly Arg Arg Tyr Arg
            20                  25                  30

Gln Arg Val Arg Asn Xaa
        35

<210>  15
<211>  27
<212>  PRT
<213>  Human

<220>
<223>  Synthetic Peptide

<220>
<221>  MOD_RES
<222>  (27)..(27)
<223>  Xaa=Leu-NH2

<400>  15

His Ser Asp Gly Ile Phe Thr Asp Ser Tyr Ser Arg Tyr Arg Lys Gln
1               5                   10                  15

Met Ala Val Lys Lys Tyr Leu Ala Ala Val Xaa
            20                  25

<210>  16
<211>  38
<212>  PRT
<213>  Human

<220>
<223>  Synthetic Peptide

<220>
<221>  MOD_RES
<222>  (38)..(38)
<223>  Xaa=Lys-NH2

<400>  16

His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr Lys
            20                  25                  30

Gln Arg Val Lys Asn Xaa
        35

<210>  17
<211>  23
<212>  PRT
<213>  Artificial Sequence

<220>
<223>  Synthetic Peptide

<220>
<221>  MOD_RES
<222>  (23)..(23)
<223>  Xaa=Leu-NH2

<400>  17

His Ser Asp Ala Val Phe Thr Asp Asn Tyr Thr Arg Leu Arg Arg Gln
1               5                   10                  15

Leu Ala Val Arg Arg Tyr Xaa
            20

<210>  18
<211>  28
<212>  PRT
<213>  Artificial Sequence

<220>
<223>  Synthetic Peptide

<220>
<221>  MOD_RES
<222>  (28)..(28)
<223>  Xaa=Asn-NH2

<400>  18

His Ser Asp Ala Val Phe Thr Asp Asn Tyr Thr Arg Leu Arg Lys Gln
1               5                   10                  15

Leu Ala Val Lys Lys Tyr Leu Ala Ala Ile Leu Xaa
            20                  25

<210>  19
<211>  30
<212>  PRT
<213>  Artificial Sequence

<220>
<223>  Synthetic Peptide

<220>
<221>  MOD_RES
<222>  (30)..(30)
<223>  Xaa=Arg-NH2

<400>  19

His Ser Asp Ala Val Phe Thr Asp Asn Tyr Thr Arg Leu Arg Arg Gln
1               5                   10                  15

Leu Ala Val Arg Arg Tyr Leu Ala Ala Ile Leu Gly Arg Xaa
            20                  25                  30

<210>  20
<211>  30
<212>  PRT
<213>  Artificial Sequence

<220>
<223>  Synthetic Peptide

<220>
<221>  MOD_RES
<222>  (1)..(1)
<223>  Xaa=Acetyl-His

<220>
<221>  MOD_RES
<222>  (30)..(30)
<223>  Xaa=Arg-NH2

<400>  20

Xaa Ser Asp Ala Val Phe Thr Asp Asn Tyr Thr Arg Leu Arg Arg Gln
1               5                   10                  15

Leu Ala Val Arg Arg Tyr Leu Ala Ala Ile Leu Gly Arg Xaa
            20                  25                  30

<210>  21
<211>  30
<212>  PRT
<213>  Artificial Sequence

<220>
<223>  Synthetic Peptide

<220>
<221>  MOD_RES
<222>  (1)..(1)

<223>  Xaa=Stearoyl-His

<220>
<221>  MOD_RES
<222>  (30)..(30)
<223>  Xaa=Arg-NH2

<400>  21

Xaa Ser Asp Ala Val Phe Thr Asp Asn Tyr Thr Arg Leu Arg Arg Gln
1               5                   10                  15

Leu Ala Val Arg Arg Tyr Leu Ala Ala Ile Leu Gly Arg Xaa
            20                  25                  30

<210>  22
<211>  30
<212>  PRT
<213>  Artificial Sequence

<220>
<223>  Synthetic Peptide

<220>
<221>  MOD_RES
<222>  (30)..(30)
<223>  Xaa=Arg-OH

<400>  22

His Ser Asp Ala Val Phe Thr Asp Asn Tyr Thr Arg Leu Arg Arg Gln
1               5                   10                  15

Leu Ala Val Arg Arg Tyr Leu Ala Ala Ile Leu Gly Arg Xaa
            20                  25                  30

<210>  23
<211>  30
<212>  PRT
<213>  Artificial Sequence

<220>
<223>  Synthetic Peptide

<220>
<221> MISC_FEATURE
<222> (17)..(17)
<223> Xaa=Nle

<220>
<221> MOD_RES
<222> (30)..(30)
<223> Xaa=Arg-NH2

<400> 23

His Ser Asp Ala Val Phe Thr Asp Asn Tyr Thr Arg Leu Arg Arg Gln
1               5                   10                  15

Xaa Ala Val Arg Arg Tyr Leu Ala Ala Ile Leu Gly Arg Xaa
            20                  25                  30

<210> 24
<211> 28
<212> PRT
<213> Artificial Sequence

<220>
<223> Synthetic Peptide

<220>
<221> MOD_RES
<222> (28)..(28)
<223> Xaa=Gly-NH2

<400> 24

His Ser Asp Ala Val Phe Thr Asp Asn Tyr Thr Arg Leu Arg Arg Gln
1               5                   10                  15

Leu Ala Val Arg Arg Tyr Leu Ala Ala Ile Leu Xaa
            20                  25

<210> 25
<211> 29
<212> PRT
<213> Artificial Sequence

<220>
<223> Synthetic Peptide

<220>
<221> MOD_RES
<222> (29)..(29)
<223> Xaa=Lys-NH2

<400> 25

His Ser Asp Ala Val Phe Thr Asp Asn Tyr Thr Arg Leu Arg Arg Gln
1               5                   10                  15

Leu Ala Val Arg Arg Tyr Leu Ala Ala Ile Leu Gly Xaa
            20                  25

<210> 26
<211> 29
<212> PRT
<213> Artificial Sequence

<220>
<223> Synthetic Peptide

<220>
<221> MOD_RES
<222> (29)..(29)
<223> Xaa=Arg-NH2

<400> 26

His Ser Asp Ala Val Phe Thr Asp Asn Tyr Thr Arg Leu Arg Arg Gln
1               5                   10                  15

Leu Ala Val Arg Arg Tyr Leu Ala Ala Ile Leu Gly Xaa
            20                  25

<210> 27
<211> 30
<212> PRT
<213> Artificial Sequence

<220>

<223>  Synthetic Peptide

<220>
<221>  MOD_RES
<222>  (30)..(30)
<223>  Xaa=Arg-NH2

<400>  27

His Ser Asp Ala Val Phe Thr Asp Asn Tyr Thr Arg Leu Arg Arg Gln
1               5                   10                  15

Leu Ala Val Arg Arg Tyr Leu Ala Ala Ile Leu Gly Lys Xaa
            20                  25                  30

<210>  28
<211>  30
<212>  PRT
<213>  Artificial Sequence

<220>
<223>  Synthetic Peptide

<220>
<221>  MOD_RES
<222>  (30)..(30)
<223>  Xaa=Arg-NH2

<400>  28

His Ser Asp Ala Val Phe Thr Glu Asn Tyr Thr Arg Leu Arg Arg Gln
1               5                   10                  15

Leu Ala Val Arg Arg Tyr Leu Ala Ala Ile Leu Gly Arg Xaa
            20                  25                  30

<210>  29
<211>  23
<212>  PRT
<213>  Artificial Sequence

<220>
<223>  Synthetic Peptide

<220>
<221> MOD_RES
<222> (23)..(23)
<223> Xaa=Leu-NH2

<400> 29

His Ser Asp Ala Val Phe Thr Glu Asn Tyr Thr Arg Leu Arg Arg Gln
1               5                   10                  15

Leu Ala Val Arg Arg Tyr Xaa
            20

<210> 30
<211> 30
<212> PRT
<213> Artificial Sequence

<220>
<223> Synthetic Peptide

<220>
<221> MOD_RES
<222> (30)..(30)
<223> Xaa=Arg-NH2

<400> 30

His Ser Asp Ala Val Phe Thr Ala Asn Tyr Thr Arg Leu Arg Arg Gln
1               5                   10                  15

Leu Ala Val Arg Arg Tyr Leu Ala Ala Ile Leu Gly Arg Xaa
            20                  25                  30

<210> 31
<211> 23
<212> PRT
<213> Artificial Sequence

<220>
<223> Synthetic Peptide

<220>
<221> MOD_RES

<222>  (23)..(23)
<223>  Xaa=Leu-NH2

<400>  31

His Ser Asp Ala Val Phe Thr Ala Asn Tyr Thr Arg Leu Arg Arg Gln
1               5                   10                  15

Leu Ala Val Arg Arg Tyr Xaa
            20

<210>  32
<211>  30
<212>  PRT
<213>  Artificial Sequence

<220>
<223>  Synthetic Peptide

<220>
<221>  MOD_RES
<222>  (30)..(30)
<223>  Xaa=Arg-NH2

<400>  32

His Ser Asp Ala Val Phe Thr Asp Asn Tyr Thr Arg Leu Arg Arg Gln
1               5                   10                  15

Leu Ala Val Arg Arg Tyr Leu Ala Ala Val Leu Gly Arg Xaa
            20                  25                  30

<210>  33
<211>  30
<212>  PRT
<213>  Artificial Sequence

<220>
<223>  Synthetic Peptide

<220>
<221>  MOD_RES
<222>  (30)..(30)
<223>  Xaa=Arg-OH

<400>  33

His Ser Asp Ala Ile Phe Thr Asp Ser Tyr Ser Arg Tyr Arg Arg Gln
1               5                   10                  15

Leu Ala Val Arg Arg Tyr Leu Ala Ala Val Leu Gly Arg Xaa
            20                  25                  30

<210>  34
<211>  30
<212>  PRT
<213>  Artificial Sequence

<220>
<223>  Synthetic Peptide

<220>
<221>  MOD_RES
<222>  (30)..(30)
<223>  Xaa=Arg-NH2

<400>  34

His Ser Asp Ala Ile Phe Thr Asp Ser Tyr Ser Arg Tyr Arg Arg Gln
1               5                   10                  15

Leu Ala Val Arg Arg Tyr Leu Ala Ala Ile Leu Gly Arg Xaa
            20                  25                  30

<210>  35
<211>  30
<212>  PRT
<213>  Artificial Sequence

<220>
<223>  Synthetic Peptide

<220>
<221>  MOD_RES
<222>  (1)..(1)
<223>  Xaa=Acetyl-His

<220>

<221>  MOD_RES
<222>  (30)..(30)
<223>  Xaa=Arg-NH2

<400>  35

Xaa Ser Asp Ala Ile Phe Thr Asp Ser Tyr Ser Arg Tyr Arg Arg Gln
1               5                   10                  15

Leu Ala Val Arg Arg Tyr Leu Ala Ala Val Leu Gly Arg Xaa
            20                  25                  30

<210>  36
<211>  38
<212>  PRT
<213>  Artificial Sequence

<220>
<223>  Synthetic Peptide

<220>
<221>  MOD_RES
<222>  (38)..(38)
<223>  Xaa=Arg-NH2

<400>  36

His Ser Asp Ala Ile Phe Thr Asp Ser Tyr Ser Arg Tyr Arg Arg Gln
1               5                   10                  15

Leu Ala Val Arg Arg Tyr Leu Ala Ala Val Leu Gly Arg Arg Tyr Arg
            20                  25                  30

Gln Arg Val Arg Asn Xaa
        35

<210>  37
<211>  38
<212>  PRT
<213>  Artificial Sequence

<220>
<223>  Synthetic Peptide

<220>
<221> MOD_RES
<222> (38)..(38)
<223> Xaa=Arg-NH2

<400> 37

His Ser Asp Ala Ile Phe Thr Asp Ser Tyr Ser Arg Tyr Arg Arg Gln
1               5                   10                  15

Leu Ala Val Arg Arg Tyr Leu Ala Ala Ile Leu Gly Arg Arg Tyr Arg
            20                  25                  30

Gln Arg Val Arg Asn Xaa
        35

<210> 38
<211> 23
<212> PRT
<213> Artificial Sequence

<220>
<223> Synthetic Peptide

<220>
<221> MOD_RES
<222> (23)..(23)
<223> Xaa=Leu-NH2

<400> 38

His Ser Asp Ala Ile Phe Thr Asp Ser Tyr Ser Arg Tyr Arg Arg Gln
1               5                   10                  15

Leu Ala Val Arg Arg Tyr Xaa
            20

<210> 39
<211> 23
<212> PRT
<213> Artificial Sequence

<220>
<223> Synthetic Peptide

<220>
<221> MOD_RES
<222> (1)..(1)
<223> Xaa=Acetyl-His

<220>
<221> MOD_RES
<222> (23)..(23)
<223> Xaa=Leu-NH2

<400> 39

Xaa Ser Asp Ala Ile Phe Thr Asp Ser Tyr Ser Arg Tyr Arg Arg Gln
1               5                   10                  15

Leu Ala Val Arg Arg Tyr Xaa
            20

<210> 40

<211> 30
<212> PRT
<213> Artificial Sequence

<220>
<223> Synthetic Peptide

<220>
<221> MOD_RES
<222> (30)..(30)
<223> Xaa=Arg-NH2

<400> 40

His Ser Asp Ala Ile Phe Thr Asp Ser Tyr Ser Arg Tyr Arg Arg Gln
1               5                   10                  15

Leu Ala Val Arg Arg Tyr Leu Ala Ala Val Leu Gly Arg Xaa
            20                  25                  30